# EUROPEAN PATENT SPECIFICATION

(11) **EP 1 539 808 B1**
(45) Date of publication and mention of the grant of the patent: **08.04.2009**
(21) Application number: 03765007.4
(22) Date of filing: 16.07.2003
(51) Int. Cl.: C07K 14/03, C12N 5/10, A61K 39/12, A61K 39/29, C07K 14/18

(54) **gM-NEGATIVE EHV-MUTANTS WITHOUT HETEROLOGOUS ELEMENTS**
GM-NEGATIVE EHV-MUTANTE OHNE HETEROLOGE ELEMENTEN
MUTANTS DU VIRUS DE L'HERPES EQUIN GRAM NEGATIFS DEPOURVUS D'ELEMENTS HETEROLOGUES

(30) Priority: 19.07.2002 DE 10233064
(43) Date of publication of application: 15.06.2005
(73) Proprietor: Boehringer Ingelheim Vetmedica GmbH, 55216 Ingelheim am Rhein (DE)
(72) Inventor: NEUBAUER, Antonie, 81545 München (DE); ZIEGLER, Christina, 81371 München (DE)
(86) International application number: PCT/EP2003/007730
(87) International publication number: WO 2004/009802

(56) References cited:
- EP-A- 1 129 722
- US-B1- 6 193 983
- SEYBOLDT C ET AL: "EQUINE HERPESVIRUS 1 (EHV-1) GYLCOPROTEIN M: EFFECT OF DELETIONS OF TRANSMEMBRANE DOMAINS" VIROLOGY, ACADEMIC PRESS,ORLANDO, US, vol. 278, no. 2, 2000, pages 477-489, XP001002104 ISSN: 0042-6822
- NEUBAUER ANTONIE ET AL: "Equine herpesvirus 1 mutants devoid of glycoprotein B or M are apathogenic for mice but induce protection against challenge infection" VIROLOGY, ACADEMIC PRESS,ORLANDO, US, vol. 239, no. 1, 8 December 1997 (1997-12-08), pages 36-45, XP002146619 ISSN: 0042-6822
- OSTERRIEDER NIKOLAUS ET AL: "The Equine Herpesvirus 1 Glycoprotein gp21/22a, the Herpes Simplex Virus Type 1 gM Homolog, is involved in Virus Penetration and Cell-to-Cell Spread of Virions" JOURNAL OF VIROLOGY, NEW YORK, US, US, vol. 70, no. 6, June 1996 (1996-06), pages 4110-4115, XP002146618 ISSN: 0022-538X
- TELFORD E A ET AL: "The DNA sequence of equine herpesvirus-4" JOURNAL OF GENERAL VIROLOGY, SOCIETY FOR GENERAL MICROBIOLOGY, READING, GB, vol. 79 ( Pt 5), May 1998 (1998-05), pages 1197-1203, XP002225299 ISSN: 0022-1317
- TELFORD E A R ET AL: "THE DNA SEQUENCE OF EQUINE HERPESVIRUS-1" VIROLOGY, ACADEMIC PRESS,ORLANDO, US, vol. 189, no. 1, 1992, pages 304-316, XP000886227 ISSN: 0042-6822

## Description

### Field of the invention

The present invention relates to the field of animal health and in particular of Equine Herpes Viruses (EHV) wherein the gene encoding the protein gM is absent, and which is free of heterologous elements. Further aspects of the invention relate to pharmaceutical compositions comprising said viruses, uses thereof, and methods for the prophylaxis and treatment of EHV infections. The invention also relates to pharmaceutical compositions comprising the combination of EHV-1 and EHV-4 viruses wherein the gene encoding the protein gM is absent and which is free of heterologous elements.

### Background of the invention

Equine herpesvirus 1 (EHV-1), a member of the *Alphaherpesvirinae,* is the major cause of virus-induced abortion in equines and causes respiratory and neurological disease. Equine herpesvirus 4 (EHV-4) can also induce respiratory symptoms, abortions or neurological disorder. The entire DNA sequence of both species (EHV-1: Strain Ab4p; EHV-4: Strain NS80567) has been determined (Telford, E. A. R. *et al.,* 1992; Telford, E. A. R. *et al.,* 1998). However, only few genes and gene products have been characterized in regard to their relevance for the virulence and immunogenic properties of EHV.
Herpesvirus glycoproteins are crucially involved in the early stages of infection, in the release of virions from cells, and in the direct cell-to-cell spread of virions by fusion of neighboring cells. To date, 11 herpes simplex virus type 1 (HSV-1)-encoded glycoproteins have been identified and have been designated gB, gC, gD, gE, gG, gH, gI, gJ, gK, gL, and gM. HSV-1 mutants lacking gC, gE, gG, gI, gJ, and gM are viable, indicating that these genes are dispensable for replication in cultured cells. Comparison of the HSV-1 and equine herpesvirus 1 nucleotide sequences revealed that all of the known HSV-1 glycoproteins are conserved in EHV-1. According to the current nomenclature, these glycoproteins are designated by the names of their HSV-1 homologs. It is known that EHV-1 gC, gE and gl are not essential for growth in cell culture, whereas gB and gD are essential for virus growth in cultured cells. The contributions of other EHV-1 glycoproteins to replication in cultured cells are not known (Flowers, C. C. *et al.;* 1992). Transcriptional and protein analyses have shown that the glycoproteins gB, gC, gD, gG, gH, and gK are expressed in EHV-1-infected cells. Glycoprotein gM (encoded by gene UL10 [Baines, J. D. *et al.,* 1991; Baines, J. D. *et al*.,1993]) is the only reported nonessential glycoprotein which is conserved in all herpesviral subfamilies and has been described for human and murine cytomegalovirus and the *Gammaherpesvirinae* members EHV-2, herpesvirus saimiri, and Epstein-Barr virus. Like many herpesvirus glycoproteins, HSV-1 gM is present in virions and membranes of infected cells. HSV-1 mutants solely lacking gM grew to titers in cell culture systems reduced approximately 10-fold relative to those of wild-type virus and showed a reduced virulence in a murine model (Baines, J. D. *et al.,* 1991; MacLean, C. A. *et al.,* 1993). The EHV-1 gM homolog (gp21/22a; refered to as EHV-1 gM from now on) was first described by Allen and Yeargan (Allen, G. P. *et al,* 1987) and was shown to be a major constituent of the virus envelope. Further investigations revealed that gene 52, the gene homologous to HSV-1 UL10, encodes the 450-amino-acid EHV-1 gM polypeptide (Pilling, A. *et al.,* 1994; Telford, E. A. R. *et al.,* 1992). EHV-1 gM represents a multiple hydrophobic protein which contains eight predicted transmembrane domains and has been reported to be present in infected cells and in purified virions as an Mᵣ 45,000 protein (Pilling, A. *et al.,* 1994; Telford, E. A. R. *et al.,* 1992).
For control of EHV-1 infections, two different approaches were followed. First, modified live vaccines (MLVs) have been developed, including the strain RacH (Mayr, A. *et al.,* 1968; Hübert, P. H. *et al.,* 1996), which is widely used in Europe and the United States. Second, inactivated vaccines and subunit vaccines based on recombinant expressed viral glycoproteins such as the glycoproteins (g) B, C, D, and H, which induced partial protection against subsequent challenge EHV-1 infection in a murine model. Subunit vaccines comprising said glycoproteins e.g. gB, gC, gD, and gH only poorly protect against reinfection (Awan et al., 1990, Osterrieder et al., 1995, Tewari et al., 1994, Stokes et al, 1996).

The technical problem underlying this invention was to provide improved vaccines which protect better against EHV infection than prior art vaccines.

### Figure legends

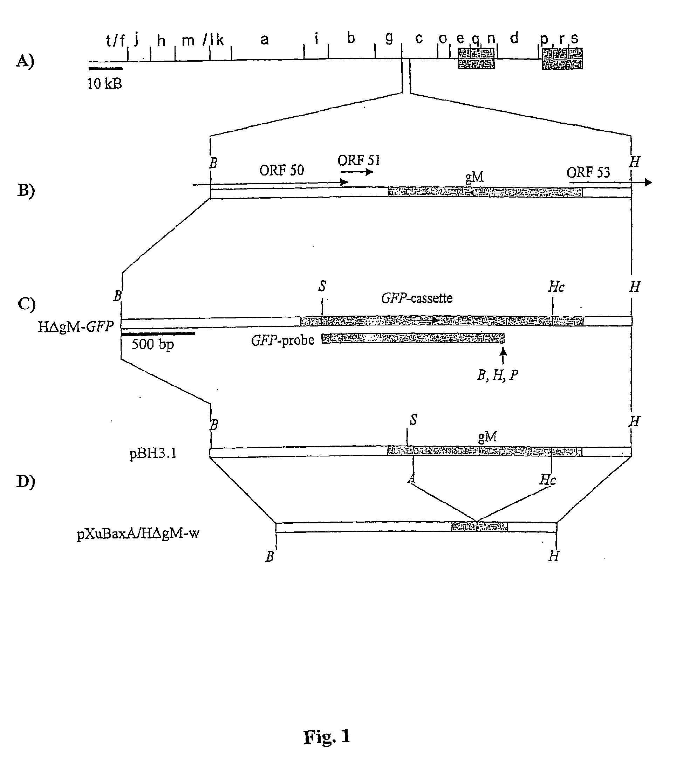

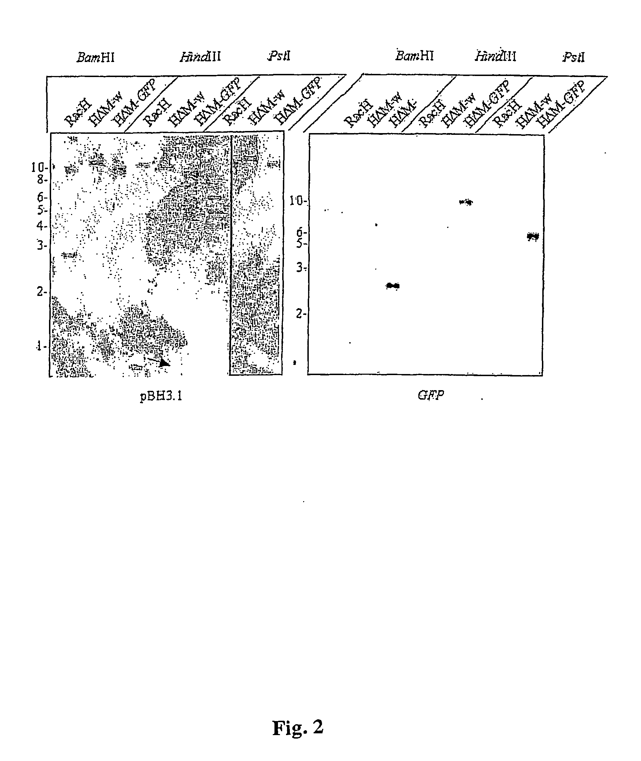

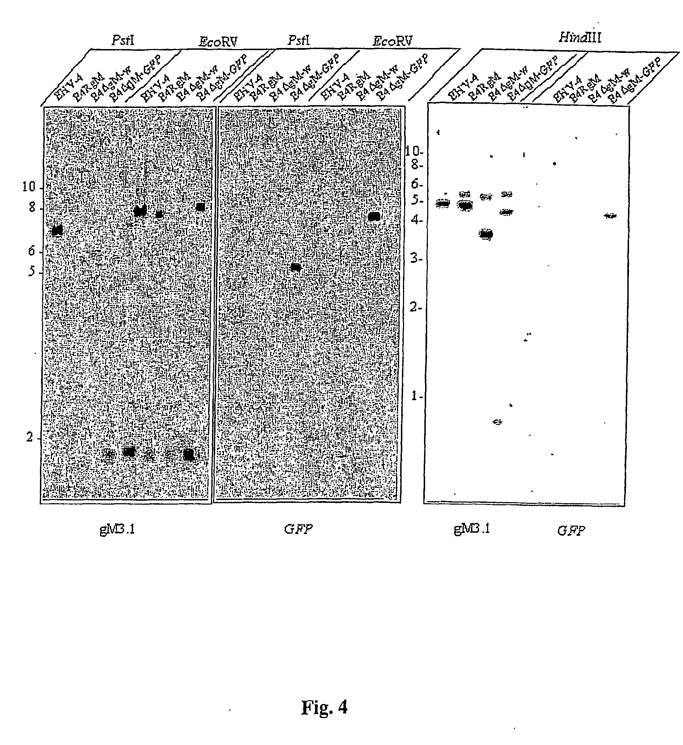

Figure 1: Generation of a gM negative EHV-1 RacH virus without foreign sequences (HΔgM-w)
   This figure shows the map of viruses and plasmids used for the construction of HΔgM-w. "First-generation" HΔgM virus has previously been constructed by either inserting the Escherichia coli lacZ (HΔgM-lacZ) or the green fluorescent protein (GFP) expression cassette (HΔgM-GFP). The BamHI map of EHV-1 strain RacH is shown (A) and the BamHI-HindIII fragment containing the gM-ORF is magnified showing the genomic organization of the region (B). The gM-negative virus, HΔgM-GFP carries a GFP-expression cassette, replacing the major part of the EHV-1 gM gene. The GFP-specific probe, that was used in Southern blots, is depicted (C). Plasmid pBH3.1 carries the EHV-1 BamHI-HindIII fragment of interest and was used to construct plasmid pXuBaxA. After cotransfection of DNA of HΔgM-GFP with plasmid pXuBaxA resulted HΔgM-w (D). Restriction sites: BamHI - B, HindIII - H, SphI - S, HincII - Hc, ApaI - A, PstI - P
Figure 2: Southern blot of gM-deleted EHV-1 virus without foreign sequences (HΔgM-w).
   DNA of RacH, HΔgM-GFP and of HΔgM-w was cleaved with BamHI, HindIII or PstI and analyzed with a GFP-specific probe (GFP) or the EHV-1 BamHI-HindIII fragment of pBH3.1 (pBH3.1). DNA-hybrids were detected by chemoluminescence using CSPD. Molecular weight marker sizes (Biolabs) are given in kbp on the left margin. The arrow points to an barely visible specific hybrid.

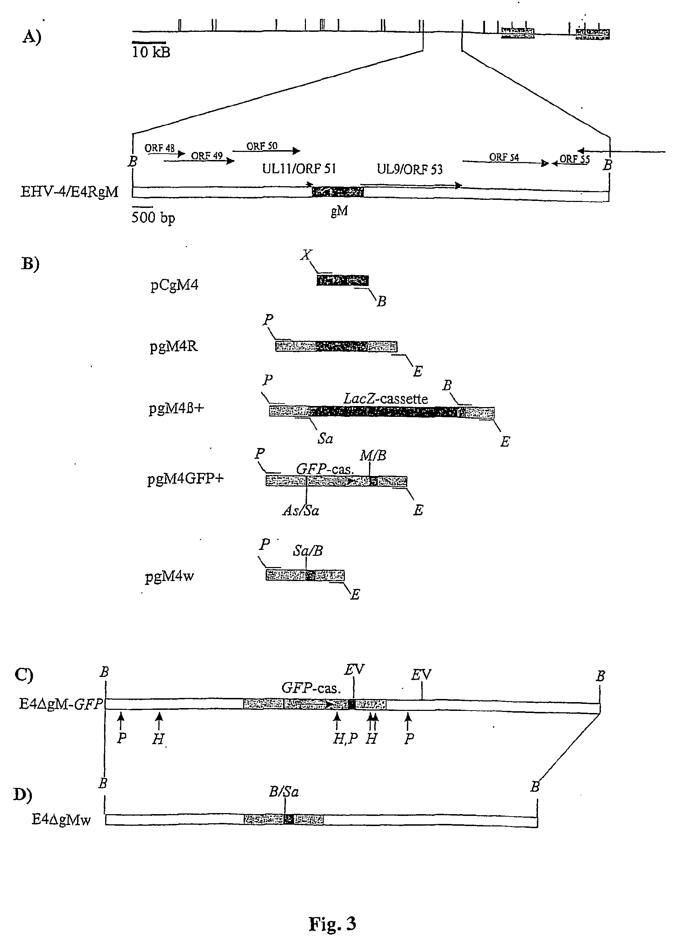

Figure 3: Generation of a gM negative EHV-4 virus without foreign sequences (E4ΔgM-w).
   In this figure, a BamHI map of EHV-4 strain NS80567 is depicted. The enlarged BamHI-e fragment encompasses the gM- and neighboring ORFs (A). Plasmid constructs and priming sites are depicted (B). Plasmid pgM4GFP+ was used for the generation of E4ΔgM-GFP, the GFP-positive and gM negative EHV-4 (B, C). Recombination of DNA of E4ΔgM-GFP with either plasmid pgM4R (B), containing 3.109 bp of EHV-4 sequences including the gM-ORF, resulted in E4RgM, the gM-repaired EHV-4 (A), or with plasmid pgM4w (B) resulted in E4ΔgM-w, the GFP- and gM-negative EHV-4 (D). Restriction sites: BamHI - B, PstI - P, EcoRl - E, Sall - Sa, Mlul - M, Asnl - As, EcoRV - EV
Figure 4: Southern blot of a gM-deleted EHV-4 virus without foreign sequences (E4ΔgM-w).
   DNA of EHV-4, E4RgM, E4ΔgM-w and E4ΔgM-GFP were cleaved with PstI, EcoRV or Hindlll as indicated and DNA-fragments blotted onto nylon membranes. Parallel membranes were either hybridized with GFP-specific sequences or with a probe, named gM3.1, containing the EHV-4 specific sequences taken out of plasmid pgM4R (Fig. 3). DNA hybrids were detected by chemoluminescence using CSPD. Molecular weight marker sizes (Biolabs) are given in kbp.

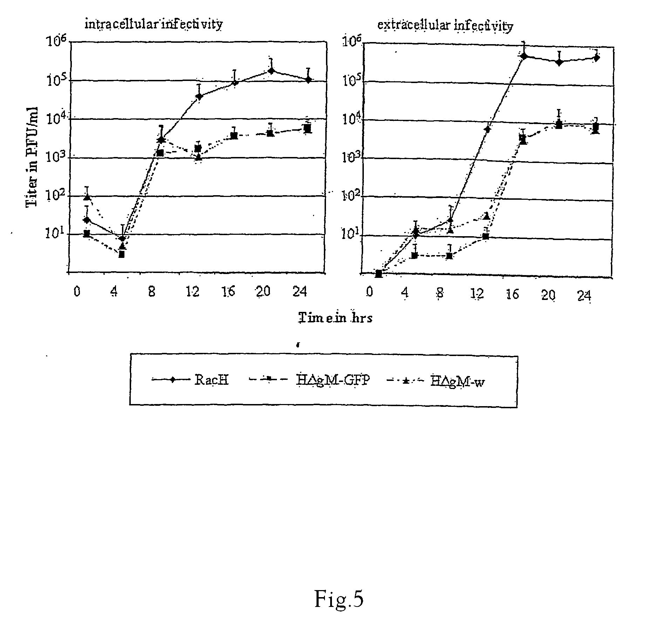

Figure 5: Growth characteristics of the gM deleted EHV-4 virus, E4ΔgM-w.
   Cells were infected with an MOI of 2 of the different viruses, as listed in the box. Kinetics of virus growth are depicted as virus titers determined in supernatants of infected cells (extracellular activity) or within infected cells (intracellular activity) relative to the time point indicated. Shown are the means of two individual experiments, standard deviations are given as error bars.

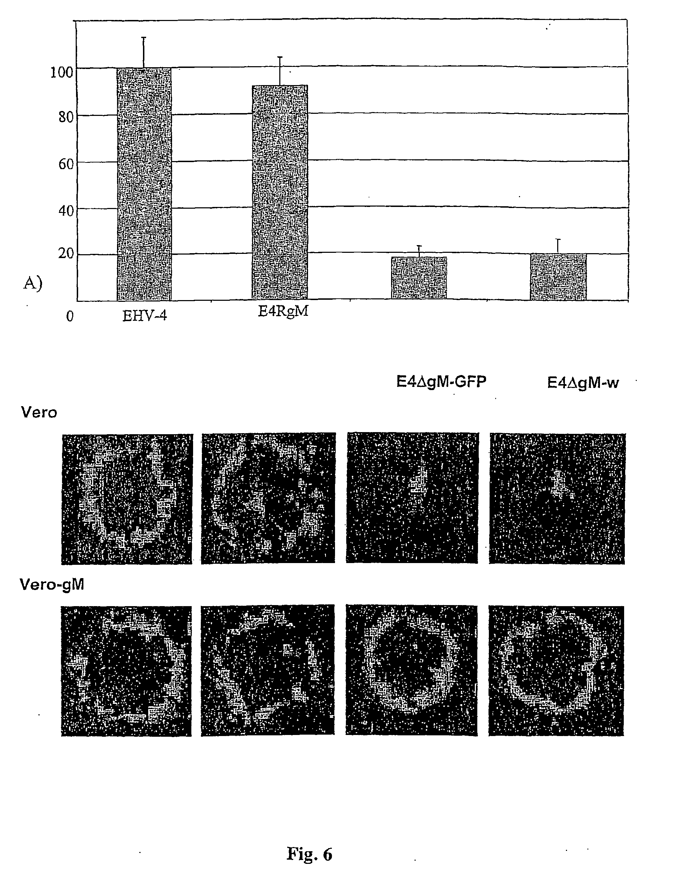

Figure 6: Plaque sizes of E4ΔM-w.
   Vero or Vero-gM cells in 6-well plates were infected with 50 PFU of either EHV-4, E4RgM, E4ΔgM-GFP or E4ΔgM-w. Maximal diameters of 150 respective plaques were determined and average plaque sizes are given in %, relative to sizes of wildtype plaques, that were set 100 %. Standard deviations are given as error bars (A). Plaques were stained by indirect immunofluorescence (anti-gD Mab 20C4, 1:1000) at day 4 p.i. and analyzed in an Axioscope (x100, Zeiss, Germany). Pictures were scanned and digitally processed (B).

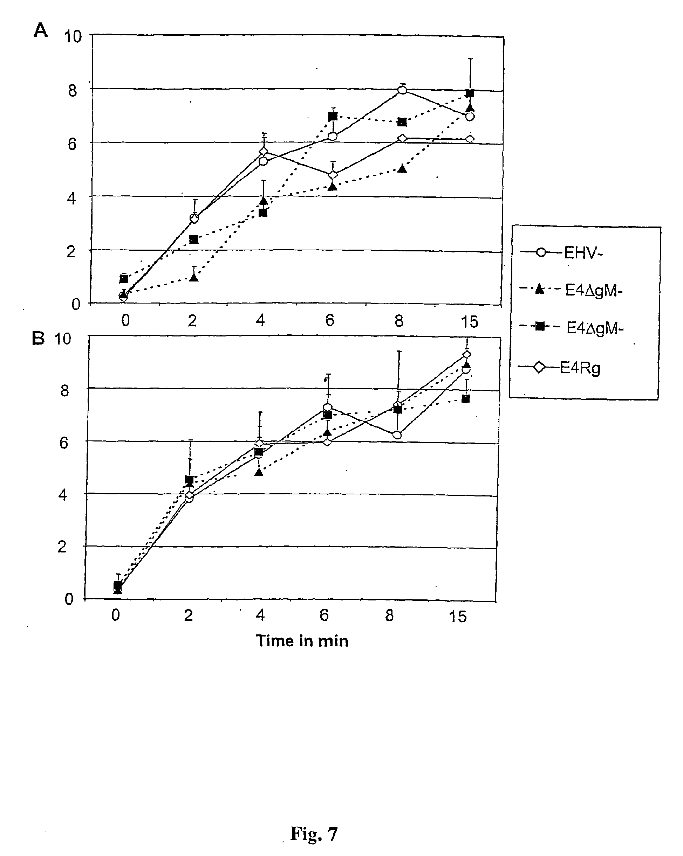

Figure 7: EHV-4 virus penetration into Vero cells.
   Penetration of EHV-4, E4RgM, E4ΔgM-w and E4ΔgM-GFP produced on non complementing Vero cells (A) or on complementing Vero-gM cells (B) into Vero cells. At given time points the penetration efficiency was determined as the percentage of the number of plaques present after citrate treatment relative to that of plaques present after control treatment. Means of two independent experiments are given. Standard deviations are depicted as error bars.

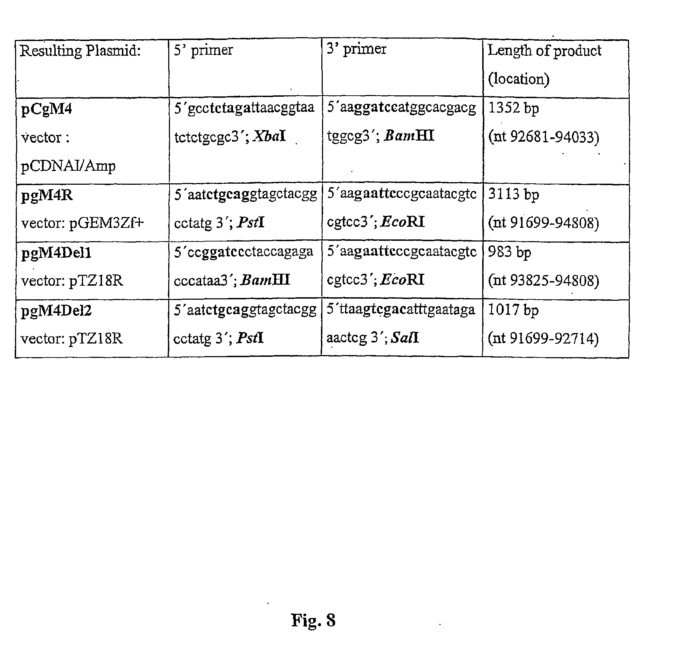

Figure 8: PCR primer sequences and location of amplificates within the EHV-4 genome.
   Sequences representing restriction enzyme recognition sites are printed in bold and the repective enzymes are listed. PCR products were inserted into the given vectors, resulting in plasmids pCgM4, pgM4R pgM4Dell or pgM4Del2, as indicated. The location of a fragment within the EHV-4 genome is given relative to the sequence determined by Telford et al. (1998).

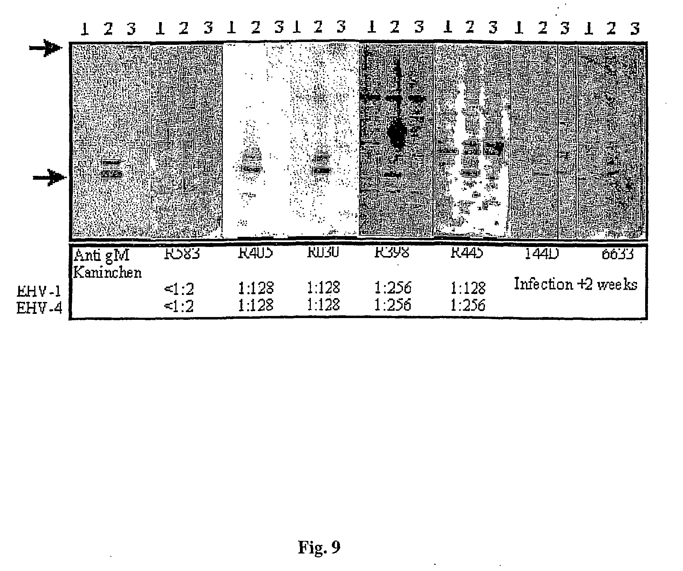

Figure 9: Western Blot analysis of horse sera.
   Lysates of EHV-1 gM expressing cell line ccgM and Rk13 cells were either heated for 2 min at 56°C (1,2) or for 5 min at 95°C (3) (gM is highly hydrophobic and known to aggregate upon boiling, so that it does not enter the separating gel anymore). Identical blots were incubated with various horse sera (1: 3000) or the anti-gM rabbit serum. Arrows point to gM specific reactivity in boiled and unboiled sampes. Neutralizing test titers (NT) are given for sera, that were obtained from the virological diagnostic unit.
Figure 10:
   (A) Comparison of EHV-1 gM to EHV-4 gM using an anti EHV-1 gM polyclonal antibody. Cells were infected with the indicated viruses (MOI of 0.5-1) and cell lysates were prepared at the stated time points p.i. EHV-1 or -4 virions were purified by repeated centrifugation through a sucrose cussion (30 %) and resuspended in PBS. Samples were mixed with buffer containing 5% 2-mercaptoethanol and then either heated to 99°C for 5 min or left at 4°C. Proteins were separated by SDS-10 % PAGE and blotted onto nitrocellulose filters. Antibody binding was visualized using anti-rabbit immunoglobulin G (IgG) peroxidase conjugate (Sigma) followed by ECLTM detection (Pharmacia-Amersham).
   (B) Virions were purified of Edmin337 cells infected with EHV-4, E4ΔgM-w, E4ΔgM-GFP or E4RgM and subjected to Western blot analysis as described in (A). gB reactivity of virions was then compared to gM reactivity using anti EHV-1 gB monoclonal antibody 3F6 or the anti EHV-1 gM polyclonal antibody.

### Disclosure of the invention

### Definitions of terms used in the description:

Before the embodiments of the present invention it must be noted that as used herein and in the appended claims, the singular forms "a", "an", and "the" include plural reference unless the context clearly dictates otherwise. Thus, for example, reference to "an EHV virus" includes a plurality of such EHV viruses comprising also all subspecies like EHV1, 4 and others, reference to the "cell" is a reference to one or more cells and equivalents thereof known to those skilled in the art, and so forth. Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications mentioned herein are incorporated herein by reference for the purpose of describing and disclosing the cell lines, vectors, and methodologies as reported in the publications which might be used in connection with the invention. Nothing herein is to be construed as an admission that the invention is not entitled to antedate such disclosure by virtue of prior invention.

The term "EHV" as used herein refers to all equine herpes viruses such as species EHV-1 and EHV-4 within the family Alphaherpesvirinae. The terms EH-virus, EH virus EHV virus, EHV-virus and EHV all relate to equine herpes viruses.

Virulence: "virulence" as used herein relates to an EH-virus capable of propagating in the target host species (i.e. horses) with the potential to induce subclinical and clinical disease characterized by respiratory symptoms, abortions or neurological disorders. Examples for virulent EHV are wild-type viruses inducing strong clinical symptoms. Examples for virulence factors are hemolysins (lysing red blood cells) or adhesins (proteins by which the pathogen can adhere to the host and initiate colonisation or invasion). More specifically, "virulence" here means that the gM gene product, a major constituent of the virus envelope, enables the virus to penetrate the host and is involved in cell-to-cell spread.

Attenuation: "An attenuated EH-virus" as used herein is relates to infectious EHV which do not cause EHV-associated subclinical or clinical disease. In particular according to the invention, such attenuated EH-viruses are EHV which can replicate and do not express gM.

A "functional variant" of the EH-virus according to the invention is EHV virus which possesses a biological activity (either functional or structural) that is substantially similar to the EHV according to the invention. The term "functional variant" also includes "a fragment", "a functional variant", "variant based on the degenerative nucleic acid code" or "chemical derivative". Such a "functional variant" e.g. may carry one or several nucleic acid exchanges, deletions or insertions. Said exchanges, deletions or insertions may account for 10% of the entire sequence. Said functional variant at least partially retains its biological activity, e.g. function as an infectious clone or a vaccine strain, or even exhibits improved biological activity.

A "variant based on the degenerative nature of the genetic code" is a variant resulting from the fact that a certain amino acid may be encoded by several different nucleotide triplets. Said variant at least partially retains its biological activity, or even exhibits improved biological activity.

A "fusion molecule" may be the DNA molecule or infectious EHV virus according to the invention fused to e.g. a reporter such as a radiolabel, a chemical molecule such as a fluorescent label or any other molecule known in the art.

As used herein, a "chemical derivative" according to the invention is a DNA molecule or infectious EHV clone according to the invention chemically modified or containing additional chemical moieties not normally being part of the molecule. Such moieties may improve the molecule's solubility, absorption, biological half life etc.

A molecule is "substantially similar" to another molecule if both molecules have substantially similar nucleotide sequences or biological activity. Thus, provided that two molecules possess a similar activity, they are considered variants as that term is used herein if the nucleotide sequence is not identical, and two molecules which have a similar nucleotide sequence are considered variants as that term is used herein even if their biological activity is not identical.

The term "vaccine" as used herein refers to a pharmaceutical composition comprising at least one immunologically active component that induces an immunological response in an animal and possibly but not necessarily one or more additional components that enhance the immunological activity of said active component. A vaccine may additionally comprise further components typical to pharmaceutical compositions. The immunologically active component of a vaccine may comprise complete virus particles in either their original form or as attenuated particles in a so called modified live vaccine (MLV) or particles inactivated by appropriate methods in a so called killed vaccine (KV). In another form the immunologically active component of a vaccine may comprise appropriate elements of said organisms (subunit vaccines) whereby these elements are generated either by destroying the whole particle or the growth cultures containing such particles and optionally subsequent purification steps yielding the desired structure(s), or by synthetic processes including an appropriate manipulation by use of a suitable system based on, for example, bacteria, insects, mammalian or other species plus optionally subsequent isolation and purification procedures, or by induction of said synthetic processes in the animal needing a vaccine by direct incorporation of genetic material using suitable pharmaceutical compositions (polynucleotide vaccination). A vaccine may comprise one or simultaneously more than one of the elements described above.
The term "vaccine" as understood herein is a vaccine for veterinary use comprising antigenic substances and is administered for the purpose of inducing a specific and active immunity against a disease provoked by EHV. The EHV vaccine according to the invention confers active immunity that may be transferred passively via maternal antibodies against the immunogens it contains and sometimes also against antigenically related organisms.
Additional components to enhance the immune response are constituents commonly referred to as adjuvants, like e.g. aluminiumhydroxide, mineral or other oils or ancillary molecules added to the vaccine or generated by the body after the respective induction by such additional components, like but not restricted to interferons, interleukins or growth factors.
A "vaccine composition or pharmaceutical compositionessentially consists of one or more ingredients capable of modifying physiological e.g. immunological functions of the organism it is administered to, or of organisms living in or on the organism. The term includes, but is not restricted to antibiotics or antiparasitics, as well as other constituents commonly used to achieve certain other objectives like, but not limited to, processing traits, sterility, stability, feasibility to administer the composition via enteral or parenteral routes such as oral, intranasal, intravenous, intramuscular, subcutaneous, intradermal or other suitable route, tolerance after administration, controlled release properties.

### Disclosure of the invention

The solution to the above technical problem is achieved by the description and the embodiments characterized in the claims.
The invention overcomes the difficulties and prejudice in the art that an equine herpes virus cannot be generated free of foreign sequences. By using the methods according to the invention, EH-viruses of superior quality for use in vaccines are provided. The central coding sequence for the protein gM is eliminated in a way that the remaining gM carboxyterminal sequences are in a different reading frame than the aminoterminal sequences. The neighboring gene for the essential protein UL9 homolog (gene 53), its orientation and overlap with the gene coding for the protein gM requires that a minimal nucleotide sequence of the gene for gM must remain to allow the expression of gene 53 and thereby retain virus viability. Therefore, an EHV according to the invention relates to EHV's that are characterized in that the gene coding for the protein gM is deleted in a way that the expression of the gene coding for the UL9 homolog (gene 53) is not affected. The term "not affected" does not relate to a certain quantity or qualitative properties of UL9 but simply means that the expression of the gene is not affected as long as said protein is expressed by the virus and present in an essentially sufficient amount for the viability of the virus.

The long lasting need in the art for a vaccine comprising recombinant equine herpesvirus 4 was satisfied by the present inventors which overcame major difficulties in the art. The EHV-1 and EHV-4 viruses according to the invention may advantageously be used for use e.g. in a vaccine.

Hence, in a first important embodiment, the invention relates to an Equine Herpes Virus (EHV) wherein the gene encoding protein gM, and therefore gM itself, is absent,
characterized in that it is free of heterologous elements. "Free of heterologous elements" means that no foreign sequence, i.e. no non-EHV sequence, such as a lacZ- or GFP-encoding cassette, is present in the coding sequence for said virus according to the invention (a so-called "white clone"). Thus, the EHV according to the invention is entirely encoded by EHV sequences. The EHV according to the invention is free of bacterial elements or nucleic acids encoding said bacterial elements. Furthermore, almost the entire coding sequence for the gM protein and therefore the encoded above-mentioned gM protein is eliminated. Thus, preferably, said EHV according to the invention is characterized in that the protein gM is absent due to deletion of the gene coding for the protein gM. However, as set out *supra,* "the gene encoding protein gM is absent" also requires that a minimum gM sequence remains so that at least the overlapping gene 53 sequence is still present, the remaining gM sequences may be deleted (see *infra*)*.* This may all be accomplished by molecular biology techniques (see *infra*) so that recombinant EHV are generated.
The use of lacZ as a marker for successful deletion of the gM gene of EHV-1 or 4 did not lead to successful generation of viruses according to the invention (see examples 1, 2). The inventors therefore developed an inventive method to obtain said virus. An EH-virus was constructed in which the gM gene was deleted by insertion of a cassette containing the GFP marker. This approach surprisingly allowed the differentiation between input virus (green fluorescent plaques) and new recombinant plaques (non-fluorescent plaques).

Preferred is an EHV obtainable by a method comprising the steps of
a) isolating a wild-type EHV;
b) establishing a plasmid encoding the EHV gM gene, optionally with flanking sequences;
c) generating a complementing cell line expressing gM or parts thereof;
d) establishing an EH virus carrying a GFP-encoding cassette insert in its gM coding sequence by co-transfecting the complementing cell line of step b) with EHV-nucleic acid and a plasmid encoding gM which is interrupted by a GFP-encoding cassette insert;
e) deleting the GFP-encoding cassette; and
f) selecting for the EHV clones wherein the GFP-encoding cassette is successfully deleted.
   "lacZ" is known to the artisan as the gene encoding β-galactosidase. According to the invention, "GFP" relates to green fluorescent protein (GFP) produced by the bioluminescent jellyfish (Chalfie et al., 1994).
   "Complementing cell line" refers to a cell line, into which a gene normally not present in the cell line genome is introduced and expressed constitutively. Useful cell lines include, but are not limited to rabbit kidney cell line Rk13, cell line cc (Seyboldt et al., 2000) or the Vero cell lines (ATCC catalogue # CRL-1586), such as clone 1008, as also disclosed in examples 1 and 2, and any other cell line known to the artisan. Usually it can be selected for cell clones expressing this additional protein. This cell line expresses the gene which is deleted in the virus, complementing this deficiency, and enables the growth of the virus after gene deletion.
   Standard molecular biology methods of use of restriction enzymes, ligation, PCR, transfection etc. are known in the art (see e.g. Sambrook et al. (1989). Molecular Cloning: A Laboratory Manual, 2nd ed., Cold Spring Harbor Laboratory Press, Cold Spring Harbor, New York
   Preferably, such EHV according to the invention is characterized in that it is EHV-1. More preferred, the EHV-1 according to the invention is characterized in that 850-1100 bp of the 1332 bp gM open reading frame are deleted. Even more preferred, the EHV-4 according to the invention is characterized in that 900-1000 bp of the gM open reading frame are deleted. More preferred also, the EHV-1 according to the invention is
characterized in that 960-970 bp of the gM open reading frame are deleted (960, 961, 962, 963, 964, 965, 966, 967, 968, 969 or 970 bp). Most preferred, the EHV-1 according to the invention is characterized in that 962 bp of the gM open reading frame are deleted.
More preferred, the EHV-1 according to the invention is characterized in that the coding sequence for gM is deleted except for 150-200 base pairs (bp) of the coding sequence encoding the C-terminal portion of gM and 150-250 bp of the coding sequence encoding the N-terminal portion of gM. In this more preferred embodiment, the coding sequence of gM is deleted, only nucleotides 93118-93267 to 93118-93317 of the sequence encoding the C-terminal portion of gM and nucleotides 94223-94472 to 94323-94472 of the coding sequence encoding the N-terminal portion of gM remain. Thus, more preferred is an EHV-1 according to the invention characterized in that nucleotides 93268-93318 to 94222-94322 (encoding the core portion of gM) are deleted (numbering according to Telford, 1992). Within the given ranges, any number of nucleotides may be deleted. Thus, according to the invention, the deletions may start no lower than nucleotide position 93268, but has to begin at position 93318. The deletion may end as early as position 94222, but no later than position 94322. Thus, a preferred EHV-1 according to the invention is characterized in that nucleotides 93268 to 94322 of the gM coding sequence as corresponding to Telford positions (1992) are deleted. Any combination is within the scope of the invention, such as 93272 to 94312, 93300 to 94300 and so forth.
Even more preferred, the EHV-1 according to the invention is characterized in that the coding sequence for gM is deleted except for 160-190 bp of the coding sequence encoding the C-terminal portion of gM and 190-220 bp of the coding sequence encoding the N-terminal portion of gM. In this more preferred embodiment, the coding sequence of gM is deleted, only nucleotides 93118-93277 to 93118-93307 of the sequence encoding the C-terminal portion of gM and nucleotides 94253-94472 to 94283-94472 of the coding sequence encoding the N-terminal portion of gM remain. Thus, more preferred is an EHV-1 according to the invention characterized in that nucleotides 93278-93308 to 94252-94282 (encoding the core portion of gM) are deleted (numbering according to Telford, 1992).
More preferred also, the EHV-1 according to the invention is characterized in that the coding sequence for gM is deleted except for 180 to 190 (180, 181, 182, 183, 184, 185, 186, 187, 188, 189, 190) bp of the coding sequence encoding the C-terminal portion of gM coding sequence and 200 to 210 (200, 201, 202, 203, 204, 205, 206, 207, 208, 209 or 210) bp of the coding sequence encoding the N-terminal portion of gM. In this more preferred embodiment, the coding sequence of gM is deleted, only nucleotides 93118-93297 to 93118-93307 (93297, 93298, 93299, 93300, 93301, 93302, 93303, 93304, 93305, 93306, 93307) of the sequence encoding the C-terminal portion of gM and nucleotides 94263-94472 to 94273-94472 (94263, 94264, 94265, 94266, 94267, 94268, 94269, 94270, 94271, 94272, 94273) of the coding sequence encoding the N-terminal portion of gM remain. Thus, more preferred is an EHV-1 according to the invention characterized in that nucleotides 94298-94308 to 94262-94272 (encoding the core portion of gM) are deleted (numbering according to Telford, 1992). This means, that any nucleotides inside the above-mentioned remaining nucleotides may be deleted according to the invention, e.g. nucleotides 94299-94263 or 94299-94264 or 94300-94272 or any combination thereof.

Most preferred, the EHV-1 according to the invention is characterized in that the coding sequence for gM is deleted except for 184 bp of the coding sequence encoding the C-terminal portion of gM coding sequence and 209 bp of the coding sequence encoding the N-terminal portion of gM. In this most preferred embodiment, the coding sequence of gM is deleted, only nucleotides 93118-93301 of the sequence encoding the C-terminal portion of gM and nucleotides 94264-94472 of the coding sequence encoding the N-terminal portion of gM remain. Thus, most preferred is an EHV-1 according to the invention characterized in that nucleotides 94263 to 93302 (encoding the core portion of gM) are deleted (numbering according to Telford, 1992). In this most preferred embodiment, 962 nucleotides of the sequence encoding gM are deleted. This is exemplified in a non-limiting manner in example 1.
Also more preferred is an EHV-1 characterized in that gM is deleted and it is free of heterologous elements and it is a recombinant variant based on a strain selected from the group of AB69 (ATCC VR2581), EHV-1 Ts-mutant ECACC V99061001, KyA, KyD, Ab1, Ab4, RacH, RacL11 or RacM of EHV-1 and no heterologous elements such as GFP- or lacZ-elements are present. Also more preferred, an EHV-1 according to the invention is characterized in that gM is deleted and it is free of heterologous elements such as GFP- or lacZ-elements and it is a recombinant variant based on on strain RacH of EHV-1. Most preferred, an EHV-1 according to the invention is characterized in that gM is deleted and it is free of heterologous elements such as GFP- or lacZ-elements and it is the RacH-based recombinant variant isolate HΔgM-w as disclosed in example 1. Said EHV-1 HΔgM-w according to the invention was deposited at the "Centre for Applied Microbiology and Research (CAMR) and European Collection of Cell Cultures (ECACC)", Salisbury, Wiltshire SP4 OJG, UK, as patent deposit according to the Budapest Treaty. The date of deposit was October 16, 2002, the preliminary identification reference is H-delta-gM-w, and the accession number given by the international depositary authority ECACC/CAMR is 02101663. Also preferred are EHV-1 having all of the identifying characteristics of said deposited EHV-1.
All before-mentioned EHV-1 have superior properties over viruses with heterologous elements such as GFP. Said EHV-1 according to the invention have an advantageously higher extracellular infectivity than those still comprising heterologous elements. This is exemplified in figure 5 (e.g. between 4 and 12 hours).
Until the present invention was made, no one in the art was able to generate a recombinant EHV-4 virus which may be used as a vaccine. EHV-1 and EHV-4 are homologous and cross-reactive to some degree. However, there was a long need in the art for attenuated EHV-4 viruses as EHV-1 does not appear to provide sufficient protection against EHV-4 infection. Thus, preferably, an EHV according to the invention is characterized in that it is EHV-4. More preferred, the EHV-4 according to the invention is characterized in that 900-1150 bp of the 1332 bp gM open reading frame are deleted. Even more preferred, the EHV-4 according to the invention is characterized in that 1000-1150 bp of the gM open reading frame are deleted. More preferred also, the EHV-1 according to the invention is characterized in that 1110-1115 bp of the gM open reading frame are deleted (1110, 1111, 1112, 1113, 1114 or 1115 bp). Most preferred, the EHV-1 according to the invention is characterized in that 1110 bp of the gM open reading frame are deleted.
More preferred, the EHV-4 according to the invention is characterized in that the coding sequence for gM is deleted except for 0-50 base pairs (bp) of the coding sequence encoding the C-terminal portion of gM and 150-250 bp of the coding sequence encoding the N-terminal portion of gM. In this more preferred embodiment, the coding sequence of gM is deleted, only nucleotides 92681-92680 to 92681-92730 of the sequence encoding the C-terminal portion of gM and nucleotides 93766-94033 to 93866-94033 of the coding sequence encoding the N-terminal portion of gM remain. Thus, more preferred is an EHV-4 according to the invention characterized in that nucleotides 92681-92731 to 93765-93865 (encoding the core portion of gM) are deleted (numbering according to Telford, 1998). Within the given ranges, any number of nucleotides may be deleted. Thus, according to the invention, the deletions may start no lower than nucleotide position 92681, but has to begin at position 92731. The deletion may end as early as position 93765, but no later than position 93865. Thus, preferably, an EHV-4 according to the invention is characterized in that nucleotides 92681 to 93865 of the gM coding sequence as corresponding to Telford positions (1998) are deleted. Any combination is within the scope of the invention, such as 92672 to 93801, 92700 to 93800 and so forth.
Even more preferred, the EHV-4 according to the invention is characterized in that the coding sequence for gM is deleted except for 10-40 bp of the coding sequence encoding the C-terminal portion of gM and 190-220 bp of the coding sequence encoding the N-terminal portion of gM. In this more preferred embodiment, the coding sequence of gM is deleted, only nucleotides 92681-92690 to 92681-92720 of the sequence encoding the C-terminal portion of gM and nucleotides 93806-94033 to 93836-94033 of the coding sequence encoding the N-terminal portion of gM remain. Thus, more preferred is an EHV-4 according to the invention characterized in that nucleotides 92691-92721 to 93805-93835 (encoding the core portion of gM) are deleted (numbering according to Telford, 1998).
More preferred also, the EHV-4 according to the invention is characterized in that the coding sequence for gM is deleted except for 30 to 40 (30, 31, 32, 33, 34, 35, 36, 37, 38, 39 or 40) bp of the coding sequence encoding the C-terminal portion of gM coding sequence and 200 to 210 (200, 201, 202, 203, 204, 205, 206, 207, 208, 209 or 210) bp of the coding sequence encoding the N-terminal portion of gM. In this more preferred embodiment, the coding sequence of gM is deleted, only nucleotides 92681-92710 to 92681-92720 (92710, 92711, 92712, 92713, 92714, 92715, 92716, 92717, 92718, 92719, 92720) of the sequence encoding the C-terminal portion of gM and nucleotides 93816-94033 to 93826-94033 (93824, 93825, 93826, 93827, 93828, 93829, 93830, 93831, 93832, 93833, 93834) of the coding sequence encoding the N-terminal portion of gM remain. Thus, more preferred is an EHV-4 according to the invention
characterized in that nucleotides 92711-92721 to 93823-93833 (encoding the core portion of gM) are deleted (numbering according to Telford, 1998). This means, that any nucleotides inside the above-mentioned remaining nucleotides may be deleted according to the invention, e.g. nucleotides 94299-94257 or 94299-94256 or 94300-94257 or any combination thereof.
Most preferred, the EHV-4 according to the invention is characterized in that the coding sequence for gM is deleted except for 34 bp of the coding sequence encoding the C-terminal portion of gM coding sequence and 209 bp of the coding sequence encoding the N-terminal portion of gM. In this most preferred embodiment, the coding sequence of gM is deleted, only nucleotides 92681-92714 of the sequence encoding the C-terminal portion of gM and nucleotides 93825-94033 of the coding sequence encoding the N-terminal portion of gM remain. Thus, most preferred is an EHV-4 according to the invention characterized in that nucleotides 92715 to 93824 (encoding the core portion of gM) are deleted (numbering according to Telford, 1998). In this most preferred embodiment, 1110 nucleotides of the sequence encoding gM are deleted. This is exemplified in a non-limiting manner in example 2.

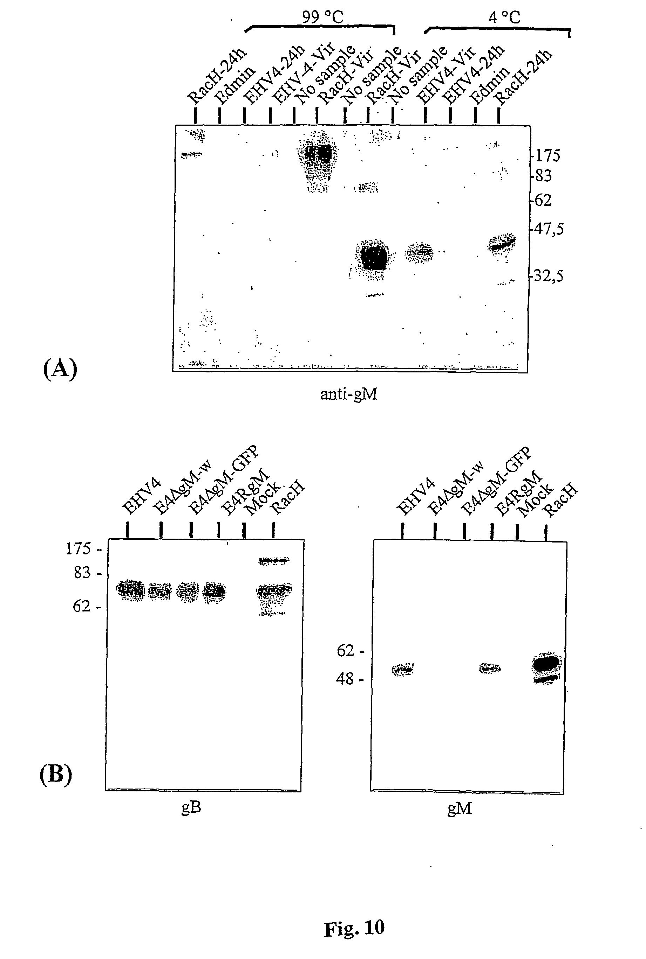

Also more preferred, an EHV-4 according to the invention is characterized in that gM is deleted and it is free of heterologous elements such as GFP- or lacZ-elements and it is a recombinant variant based on strain MSV Lot 071398 of EHV-4. Most preferred, an EHV-4 according to the invention is characterized in that gM is deleted and it is free of heterologous elements such as GFP- or lacZ-elements and it is based on strain MSV Lot 071398 and isolate E4ΔgM-4 as disclosed in example 2. Said EHV-1 HΔgM-w according to the invention was deposited at the "Centre for Applied Microbiology and Research (CAMR) and European Collection of Cell Cultures (ECACC)", Salisbury, Wiltshire SP4 OJG, UK, as patent deposit according to the Budapest Treaty. The date of deposit was January 14, 2003, the preliminary identification reference is EHV-4, and the accession number given by the international depositary authority ECACC/CAMR is 03011401. Also preferred are EHV-4 having all of the identifying characteristics of said deposited EHV-4.
All before-mentioned EHV-4 have superior properties over viruses known in the prior art as there are no recombinant EHV-4 available in the art. Furthermore, said EHV-4 according to the invention have an advantageously higher extracellular infectivity than those still comprising heterologous elements such as GFP. This is exemplified in figure 10 (e.g. at 24 hours).

Another important element of the invention is a nucleic acid coding for an EHV as disclosed supra. The artisan can easily determine the corresponding sequence by standard molecular biology methods known in the art.

There was a particular difficulty in the art to obtain the EHV according to the invention. The present inventors constructed gM negative EHV viruses introducing a marker gene (lacZ) into the gM gene. When it was attempted to remove this cassette, in both EHV-1 and EHV-4 mutants produced by lacZ insertion all clones being phenotypically lacZ negative still contained the lacZ cassette. The inventors therefore developed an inventive method to obtain said viruses. An EH virus was constructed in which the gM gene was deleted by insertion of a cassette containing the GFP marker. This approach surprisingly allowed the differentiation between input virus (green fluorescent plaques) and new recombinant plaques (non-fluorescent plaques). Also, a Vero cell line (based on Vero cell clone 1008) constitutively expressing EHV4-gM was generated by the present inventors to overcome the difficulties in the art. Said cell line was generated by transfection of the appropriate gM gene and subsequent selection for gM-expressing Vero cells. Only said cells enabled the inventors to replicate EHV4 gM negative virus.

Said gM-complementing Vero cell line according to the invention was deposited at the "Centre for Applied Microbiology and Research (CAMR) and European Collection of Cell Cultures (ECACC)", Salisbury, Wiltshire SP4 0JG, UK, as patent deposit according to the Budapest Treaty. The date of deposit was January 28, 2003, the preliminary identification reference is VERO GM, and the accession number given by the international depositary authority ECACC/CAMR is 03012801. Also preferred are cell lines having all of the identifying characteristics of said deposited VERO GM cell line.

Preferred is a method for obtaining a recombinant EHV, comprising the steps of
a) isolating a wild-type EHV;
b) establishing a plasmid encoding the EHV gM gene, optionally with flanking sequences;
c) generating a complementing cell line expressing gM or parts thereof;
d) establishing an EH virus carrying a GFP-encoding cassette insert in its gM coding sequence by co-transfecting the complementing cell line of step b) with EHV-nucleic acid and a plasmid encoding gM which is interrupted by a GFP-encoding cassette insert;
e) deleting the GFP-encoding cassette; and
f) selecting for the EHV clones wherein the GFP-encoding cassette is successfully deleted.

Said above-captioned cells are an important embodiment of the present invention. Thus, the invention relates to a cell line for use in a method according to the invention, characterized in that the gene encoding the protein gM is transfected into said cell line and said cell line expresses gM. The invention preferably relates to a cell line according to the invention, characterized in that it is a cell line selected from the group of Vero cells (Vero-gM cells), RK-13, and cc (cc-gM).
As disclosed *supra* for EHV-1, the use of lacZ as a marker instead of GFP did also in EHV-4 not lead to successful generation of viruses according to the invention (see in a non-limiting manner in example 2). "LacZ-positive" cells generally stained less intense on Vero cells than on Rk13 cells and were thus harder to identify, and the EHV-4 system replicated slower than EHV-1 and thus gave less time between plaque identification and isolation of viable virus progeny. Therefore, the use of GFP represented the only way to obtain said EHV-4 virus. The procedure was carried out as described *supra* for EHV-1 and surprisingly also led to the successful identification of EHV-4 gM deleted virus by virtue of identifying fluorescent plaques.

The isolation of wild-type EHV is accomplished by collecting lung tissue at necropsy from animals suspected to have been diseased by EHV, and isolating EHV on tissue cells as known in the art. The EHV 1 complete genome sequence has been published by Telford et al. (1992). Likewise, the complete genome sequence for EHV-4 has been published by Telford et al. (1998). The PCR amplification of DNA sequences by use of specific primers binding to complementary strands of target DNA flanking the DNA stretch of interest represents a standard molecular biology method. Methods for ligating appropriate DNA sequences into plasmids suitable for the constructions intended, for DNA transfection into eukaryotic cells, for Southern Blot and Western Blot analyses, for site-directed excision of DNA fragments via restriction enzymes and for selection of cell lines expressing the desired heterologous gene or plasmids harboring the desired gene or virus in which a certain gene is deleted are known to the skilled person. Standard molecular biology methods such as above mentioned techniques are known to the skilled person and can also be found e.g. in Sambrook et al.(1989) Molecular Cloning: A Laboratory Manual, 2nd ed., Cold Spring Harbor Laboratory Press, Cold Spring Harbor, New York and Bertram, S. and Gassen, H.G. Gentechnische Methoden, G. Fischer Verlag, Stuttgart, New York, 1991).
"Deletion" means the removal of one or several nucleotides or amino acids.

Another important embodiment of the invention is a pharmaceutical composition or vaccine comprising an EHV according to the invention, optionally in combination with a pharmaceutically acceptable carrier or excipient.

Also an important part of the present invention is a pharmaceutical composition comprising a nucleic acid according to the invention as disclosed *supra.*

Preferably, a vaccine according to the invention refers to a vaccine as defined above. The term "live vaccine" refers to a vaccine comprising a particle capable of replication, in particular, a replication active viral component.

Preferably, a vaccine according to the invention comprises a gM-deleted EHV-1 according to the invention as disclosed *supra* combined with an gM-deleted EHV-4 according to the invention as disclosed *supra* or optionally any other antigenetic group and optionally a pharmaceutically acceptable carrier or excipient. Said vaccine may be administered as a combined vaccine at the same point in time. Most preferably, said attenuated EHV-1 according to the invention may be administered first followed by an administration of an attenuated EHV-4 according to the invention three to four weeks later. Most preferably also, said attenuated EHV-1 according to the invention may be administered in combination with an attenuated EHV-4 according to the invention in a typical vaccination scheme where two or three basic vaccinations are given. A typical vaccination scheme of such a vaccine is two vaccinations four weeks apart (basic vaccination), followed by regular boosts every six months. However, any of said vaccines according to the invention as disclosed supra may also be administered at different intervals, e.g. every three months.

The artisan may choose to divide the administration into two or more applications, which may be applied shortly after each other, or at some other predetermined interval ranging. Preferably, such interval may be: 1 ° immunization, 2° immunization approx. 4 weeks thereafter and optionally 3° immunization 5-6 months thereafter. Depending on the desired duration and effectiveness of the treatment, vaccines may be administered once or several times, also intermittently. The vaccines according to the invention may be administered to a mare prior to breeding and again during its pregnancy to prevent EHV-associated abortions. Other horses can be vaccinated, e.g. once a year. Foals may be vaccinated shortly after birth.

The vaccines of the present invention may be applied by different routes of application known to the expert, notably intravenous injection or direct injection into target tissues. For systemic application, the intravenous, intravascular, intramuscular, intraarterial, intraperitoneal, oral, or intramucosal (e.g. nasal or respiratory spray or injection) routes are preferred. A more local application can be effected subcutaneously, intracutaneously, intrapulmonarily or directly in or near the tissue to be treated (connective-, bone-, muscle-, nerve-, epithelial tissue). A vaccine composition according to the invention can also be administered via an implant or orally. Most preferred is the intramuscular adminstration.

For preparing suitable vaccine preparations for the applications described above, the expert may use known injectable, physiologically acceptable sterile solutions. For preparing a ready-to-use solution for parenteral injection or infusion, aqueous isotonic solutions, such as e.g. saline or corresponding plasma protein solutions are readily available. The vaccine preparations may be present as lyophylisates or dry preparations, which can be reconstituted with a known injectable solution directly before use under sterile conditions, e.g. as a kit of parts. The final preparation of the vaccine preparations of the present invention are prepared for injection, infusion or perfusion by mixing purified virus according to the invention with a sterile physiologically acceptable solution, that may be supplemented with known carrier substances or/and excipient. The applied dose of each EH-virus according to the invention present in the vaccine formulation preferably may be between 10⁴ and 10⁸ TCID₅₀/ per animal, between 10⁵ and 10⁷ TCID₅₀/per animal, most preferably 10⁶ TCID₅₀/ per animal.

The invention further relates to the use of EHV according to the invention in the manufacture of a medicament for the prophylaxis and/or treatment of EHV-associated conditions.

The invention further relates to the use of a nucleic acid according to the invention in the manufacture of a medicament for the prophylaxis and/or treatment of EHV-associated conditions.

The invention further relates to a method for the prophylaxis and/or treatment of an animal characterized in that a pharmaceutical composition according to the invention is applied to said animal and the therapeutic success is monitored.

The invention preferably relates to a method of treating an EHV-infected equine animal with a gM-deleted EHV according to the invention as described *supra,* wherein the said attenuated EHV or the vaccine composition as disclosed *supra* is administered to the equine animal in need thereof at a suitable dosis as known to the skilled person and the reduction of EHV symptoms such as viremia and leukopenia and/or coughing and/or pyrexia and/or nasal discharge and/or diarrhea and/or depression and/or abortion is monitored. Said treatment preferably may be repeated. Thus, the invention relates to a method for the prophylaxis and/or treatment of an animal characterized in that a pharmaceutical composition according to the invention is applied to said animal and the therapeutic success is monitored. The treatment may be carried out as disclosed for the vaccine composition *supra.*

The invention preferably relates to a method of detecting antibodies against specific structures of infecting EHV-1 or EHV-4 and to a method of differentiating wild-type infections from the presence of gM deleted EHV-1 or EHV-4 as described above by an immunological method. Immunological methods are known to the expert in the field and include, but are not limited to ELISAs (***e**nzyme-**l**inked **i**mmuno-**s**orbent assay*) or *Sandwich-*ELISAs*, dot-blots,* immunoblots, radioimmunoassays (***R**adioimmuno**a**ssay RIA*), diffusion-based Ouchterlony tests, rocket immunofluorescent assays or Western-blots. Examples for immunological methods are e.g. described in: An Introduction to Radioimmunoassay and Related Techniques, Elsevier Science Publishers, Amsterdam. The Netherlands (1986); Bullock et al., Techniques in Immunocytochemistry, Academic Press, Orlando, FL Vol. 1 (1982), Vol. 2 (1983), Vol. 3 (1985); Tijssen, Practice and Theory of Enzyme Immunoassays: Laboratory Techniques in Biochemistry and Molecular Biology, Elsevier Science Publishers, Amsterdam, The Netherlands (1985). Said ELISA may use, but not be confined to the use of immobilized gM gene product or a part of gM gene product or any other EH virus 1 or EH virus 4 gene product on a plastic surface suitable for ELISA analysis.

An ELISA according to the present invention comprises, but is not limited to the steps of
a) immobilizing a gM gene product or a fragment thereof onto a plastic support
b) rinsing the plastic surface with an appropriate washing buffer (e.g., PBS-Tween)
c) adding the samples to selected wells and incubating the ELISA plate according to standardized methods
d) washing the wells of the ELISA plate and adding a suitable antibody coupled to an enzyme such as HRP (horse radish peroxidase)
   detecting bound antibody/HRP conjugate by adding a suitable substrate, followed by photometric read-out of optical density of individual wells. Suitable antibodies, e.g. rabbit anti horse lg, are known in the field.
   The following examples serve to further illustrate the present invention; but the same should not be construed as limiting the scope of the invention disclosed herein.

### Examples

### Example 1: gM deleted EHV-1 isolates

The gM negative EHV-1 were constructed by either inserting the Escherichia coli lacZ (HΔgM-lacZ) or the green fluorescent protein (GFP) expression cassette (HΔgM-GFP) thereby replacing 74.5 % of gM-gene sequences. The expression of a marker protein facilitates the identification and subsequent purification of a recombinant virus. To avoid the presence of any "non-EHV-1" sequences within the vaccine virus, it was decided to remove the marker gene sequences and construct another, second generation gM-negative EHV-1, a "white" HΔgM (HΔgM-w).
To this end, plasmid pXuBaxA was constructed (Figure 1). At first recombination of pXuBaxA-sequences into the /*acZ*-marked virus HΔgM-*lacZ* was attempted. In a first step, DNA transfections mediated by the calcium phosphate method were optimized, such, that several white plaques resulted after plating of 100-1000 PFU of transfection supernatants. Consequently, several plaques were chosen for purification of progeny virus and subjected to four to five rounds of isolation of single plaques.
Multiple, independently isolated, phenotypically *lacZ*-negative virus populations were genotypically analyzed by Southern blotting and turned out to still carry sequences of the lacZ-cassette. Difficulties in isolating truly /*acZ*-negative virus populations due to "*lacZ*-silencing" had been anticipated and therefore a great number of phenotypically *lacZ*-negative virus populations were purified and analyzed without success. Therefore, the strategy of generating a "white" gM-negative RacH virus was changed by switching to cotransfections with the gM negative EHV-1, that had been constructed by insertion of a *GFP* cassette. Using the *GFP*-expressing virus facilitated the distinction between input virus (green fluorescent plaques) and new recombinant viruses (non fluorescent plaques) and thus increased the efficiency of isolating phenotypically *GFP*-negative plaques. Changing the "input" gM-negative RacH was not supposed to influence the genotype of the expected recombinant virus as (i) both the first generation HΔgM viruses are, apart from the marker, genetically identical and as (ii) the final genotype in the region of interest is determined by the recombination plasmid (pXuBaxA).
For construction of plasmid pXuBaxA (construct necessary to obtain the "white" gM negative EHV-1) the 962 bp *Apa*I *- Hinc*II fragment within the 1352 bp open reading frame of EHV-1 gM was removed of plasmid pBH3.1 (Figure 1 D). Plasmid pBH3.1 carries the EHV-1 *Bam*HI*-Hind*III fragment surrounding the gM gene (Seyboldt et al., 2000). To prevent expression of any truncated gM-product, restriction endonucleases *Apa*I and *Hinc*II have been chosen such that after blunt end adjusting and ligation the remaining C-terminal gM sequences (183 bp) were not in frame with the remaining N-terminal sequences (208 bp).
EHV-1 gM expressing cell line ccgM (Seyboldt at al., 2000; obtained from Dr. N. Osterrieder) was maintained in minimal essential medium supplemented with 5 - 10 % fetal calf serum (Biochrom, γ-irradiated). Homologous recombination into EHV-1 was achieve by calcium phosphate mediated co-transfection of ccgM-cells with 5-10 µg of plasmid pXuBaxA (Figure 1 D) and 2 µg of DNA of HΔgM-*lacZ* or HΔgM-*GFP*, respectively.
Subsequent analysis of DNA of HΔgM-GFP with a digoxigenin labeled probe specific for the BamHI-HindIII fragment of plasmid pBH3.1 (Figure 2) revealed
(i) a 2.757 bp and a 9.043 bp fragment on BamHI,
(ii) a 10.006 bp and a 825 bp fragment on HindIII,
(iii) and to a 5.415 bp and a 4.474 bp fragment on PstI digested DNA.
   The restriction enzymes used (BamHI, HindIII, PstI) do cut within sequences of the GFP-marker cassette, thereby altering the fragment pattern relative to the respective GFP-marker cassette free DNA.
   The GFP-probe bound to the respective first fragments (i-iii) and did not detect GFP-specific sequences on DNA of RacH or of HΔgM-w.
   On DNA of RacH the expected BamHI (11.166 bp), HindIII (10.199 bp) and PstI (9.257 bp) fragments were detected, which decreased in size after removal of 962 bp of gM-sequences accordingly (to: 10.204 bp, 9.237 bp and 8.279 bp; figure 3B).
   Single-step growth kinetics of gM-negative viruses (HΔgM-w or HΔgM-GFP), which had been constructed as described in the legend to Figure 1, and RacH were performed. Rk13 cells in 24 well plates were infected at an MOI of 2 of the respective viruses. Supernatants and infected cell pellets were harvested separately at various times p.i. (0, 4, 8, 12, 16, 20, 24 h p.i.). Supernatants were cleared of cellular debris by low speed centrifugation and cells were subjected to freeze-thawing before cell-associated infectivity was assayed. All virus titers were determined individually on Rk13 or ccgM cells, respectively, in 24 well plates. The results (data not shown) can be summarized as follows: Cell-associated infectivity of both HΔgM-viruses was reduced between factor 1.6 (4 h p.i.) and 45 (20 h p.i.) on Rk13 cells when compared to titers of cells infected with RacH (intracellular infectivity). Extracellular virus titers of both the HΔgM viruses were maximally reduced by 186 (HΔgM-w) or 650 (HΔgM-GFP) fold (12 h p.i.) compared to those of RacH (extracellular infectivity), supporting a role of gM in virus egress of RacH also.

### Example 2: gM deleted EHV-4 isolates

To parallel the construction of gM-negative EHV-1, *lacZ* selection was chosen for selection of EHV-4. To allow the isolation of a gM-negative EHV-4, a Vero cell line constitutively expressing EHV-4 gM was constructed. Vero cell clone C1008 (ATCC number: CRL-1586was maintained in minimal essential medium supplemented with 5 - 10 % fetal calf serum (Biochrom, γ-irradiated). Recombinant cell line Vero-gM was generated by Effectene^{™} (Qiagen) mediated transfection of 1 µg of plasmid pCgM4 (Figure 3B) and 0.1 µg of plasmid pSV2neo (conferring resistance to G418; Neubauer et al., 1997) into Vero cells. Cell clones were first selected for resistance to G418 (Calbiochem), then for trans-complementation of a gM negative EHV-4. G418 was added to the medium of every 5^{th} passage of recombinant cell lines (500µg/ml). All cells were regularly analyzed for Mycoplasma by PCR and for Bovine Viral Diarrhoe Virus (BVDV) antigen by FACS analysis. The selected cell clone was called Vero-gM and used in the following experiments.
EHV-4 DNA was cotransfected with plasmid pgM43+ (Figure 3B) into Vero-gM cells. The recombination resulted in several "*lacZ*-positive" plaques, that were isolated and replated. But then the purification of a deletion mutant in EHV-4 turned out to be more difficult and slower than in EHV-1 as: (i) "*lacZ*-positive" plaques generally stained less intense on Vero cells than on Rk13 cells and were thus harder to identify, and as (ii) the EHV-4 system replicated slower than EHV-1 and thus gave less time between plaque identification and isolation of viable virus progeny.
All /*acZ*-positive plaques, that were isolated, were lost over the first round of purification, which made it imperative to search for another solution. It was therefore attempted to use the *GFP*-marker for EHV-4 also. In a first step, plasmid pgM4GFP+ (Figure 3B) was constructed and used for recombination into EHV-4 DNA. Resulting *GFP*-positive plaques were purified by three rounds of isolating single plaques on cell line Vero-gM. A homogenous *GFP*-positive virus population was chosen and viral DNA subjected to Southern blot analysis (Figure 4). DNA of the resulting virus, E4ΔgM-*GFP* (Figure 3C), was then cotransfected with either plasmid pgM4R or pgM4w (Figure 3B). Plasmid pgM4R was used for the construction of an gM-repaired EHV-4, called E4RgM (Figure 3A). Plasmid pgM4w was the basis to generate the simultaneously gM- and marker gene-negative EHV-4, E4ΔgM-w (Figure 3D). E4RgM and E4ΔgM-w virus populations were isolated for a *GFP*-negative phenotype, purified on Vero or on Vero-gM cells and finally analyzed by Southern blot (Figure 4). To express EHV-4 gM in eurcaryotic cells plasmid pCgM4 was generated (Figure 3B). The complete ORF of EHV-4 gM was amplified by PCR using primers given in Figure 8 and the Taq polymerase (MBI-Fermentas). The resulting PCR-product was inserted into vector pcDNAI/Amp (Invitrogene).

Plasmid pgM4R resulted after PCR-amplification of nucleotides 91.699 to 94.808 (Telford et al., 1998) of EHV-4 and insertion of the amplification product into vector pGEM3Zf+ (Promega) (Figure 3B; Figure 8). This plasmid was used for the construction of the gM-repaired EHV-4, E4RgM (Figure 3A).
To construct plasmid pgM4β+ (Figure 3B), that was designed to initially delete 1110 bp of the 1352 bp EHV-4 gM, a multistep strategy was chosen. In a first step, both the flanking sequences necessary for DNA recombination were amplified independently by PCR using the PFU polymerase (Stratagene). Restriction sites necessary for stepwise cloning were added by primer sequences (Figure 8). PCR products were inserted into vector pTZ18R (Pharmacia), resulting in plasmids pgM4Del1 and pgM4Del2 (Figure 8). In a next step the 3.9 kbp *E-coli lacZ* expression cassette was released from plasmid ptt264A+ (Osterrieder et al., 1996) by *Sal*I and *Bam*HI digest and was inserted into plasmid pgM4Del1 resulting in plasmid pgM4Del1β+. Then, EHV-4 specific sequences, taken out of pgM4Del2, were introduced into pgM4Del1β+ using *Pst*I and *Sal*I*,* such that the marker gene cassette was flanked by EHV-4 specific sequences in the resulting plasmid pgM4β+ (Figure 3B).
Plasmid pgM4β+ was then digested with *Sal*I and *Bam*HI, again releasing the *lacZ-*cassette, the resulting 5'-overhangs were filled in with the Klenow polymerase and the construct resulting from religation was called pgM4w (Fig. 3B). Again a frameshift between the N-terminal 208 nt and the remaining C-termial 33 nt of the gM-sequence was designed.
To generate plasmid pgM4GFP+ (Fig. 3B), the /*acZ*-cassette was taken out of pgM4β+ (Figure 3B) using the *Sal*I and *Bam*HI sites and replaced by the GFP-cassette (including CMV-promoter and SV40-polyA), that had been removed of vector pEGFP-C1 (Clontech) via *Asn*I and *Mlu*I digest. Restriction enzyme generated overhangs were blunt end adjusted by the Klenow polymerase.
Correct amplification of all PCR products was confirmed by cycle sequencing (MWG Biotech) and comparison to the published sequence of EHV-4 strain NS80567 (Telford et al., 1998).
Recombination into EHV-4 was done by using the cell line Vero-gM and either plasmid pgM4β+ or pgM4GFP+ (Figure 3B) was cotransfected with EHV-4 DNA to generate a first generation gM-negative EHV-4 (Figure 3C). Plasmid pgM4w (Figure 3B) in combination with DNA of the gM-negative EHV-4 resulted in E4ΔgM-w (Figure 3D). Finally, the gM-repaired EHV-4, E4RgM (Figure 3A), was isolated after co-transfection of plasmid pgM4R (Figure 3B) with DNA of E4ΔgM-*GFP* into Vero-cells.
DNA of EHV-4, E4RgM, E4ΔgM-w and E4ΔgM-*GFP* were cleaved with *Pst*I, *Eco*RV or *Hind*III and DNA-fragments blotted onto nylon membranes. Parallel membranes were either hybridized with GFP-specific sequences (identically to Figure 2) or with a probe, named gM3.1, containing the EHV-4 specific sequences taken out of plasmid pgM4R (Figure 3B).

Digestion results obtained (Figure 4) were as follows:
(i) On DNA of E4ΔgM-GFP the GFP-probe recognized fragments at 5.531 bp, when Pstl cleaved, at 8.383 bp after EcoRV-digest and at 4.528 bp after HindIII digest. Identical fragments plus fragments at 1.792 bp (PstI), 1.801 bp (EcoRV) and 826 plus 5.487 bp (HindIII) reacted with probe gM3.1.
(ii) Neither parental EHV-4, nor the repaired virus E4RgM or E4ΔgM-w carried any GFP specific sequences.
(iii) The gM3.1 reactive DNA-fragments in EHV-4 and E4RgM were detected at 6.806 bp (PstI), at 7.874 bp plus 1.801 bp (EcoRV) and at 4.837 plus 5.487bp (HindIII), respectively.
(iv) The gM- and GFP-cassette negative virus E4ΔgM-w, did not hybridize with GFP-sequences, but with the respective EHV-4 specific sequences (gM3.1). The latter probe detected fragments, lacking 1110 bp of gM sequences, when compared to wild type virus, i.e. at 5.696 bp (PstI), at 6.764 bp plus 1.801 bp (EcoRV) and at 3.727 bp plus 5.487 bp (HindIII), respectively.

On Hindlll cleaved DNA of all of these viruses another gM3.1 probe specific fragment exists at 126 bp (Fig. 2C), but this fragment is to small to be shown in this Southern blot.
Viruses E4ΔgM-GFP and E4ΔgM-w lost their capacity to express gM and in addition E4ΔgM-w lost the marker gene sequence.

a) Virus growth on culture cells. Virus growth properties of the various mutant viruses as detailed above were compared on Vero and on Vero-gM cells. Cells seeded in 24 well plates were infected at an MOI of 1-2 and extracellular (extracellular infectivity) and intracellular (intracellular infectivity) virus titers were determined at different time points p.i. (Fig. 5). While growth properties of the rescuant E4RgM virus corresponded well to those of EHV-4, there was a surprising inhibition in the production of E4ΔgM-w and E4ΔgM-GFP extracellular virus titers on non-complementing cells. Within this series of experiments (mean of two independent experiments) extracellular infectivity could never be detected before 24 h p.i.. Even at 30 hours p.i. only very low titers were extracellularly observed (maximum of 1.5 plaques/ml at the lowest dilution 10⁻¹), although cells showed severe cytopathic effect. Differences in intracellular infectivity, however, never reached 100 fold and peaked at 24 hours p.i. (84 fold between EHV-4 and E4ΔgM-w). The delay in detecting intracellular infectivity was only one time point (12 h versus 15 h. p.i.). Taken together it could be surprisingly demonstrated that deletion of gM-sequences of the EHV-4 background massively influenced virus replication in vitro, but that expression of gM is not essential for replication. Especially extracellular infectious virus decreased and the ability to directly infect adjacent cells was diminished - as reflected by plaque sizes.

b) Plaque size. Diameters of 150 plaques after infection of Vero or Vero-gM cells with EHV-4, E4RgM, E4ΔgM-w or E4ΔgM-GFP were measured and average plaque sizes were determined relative to sizes of wildtype plaques, that were set 100 %. It was clearly demonstrated, that the gM negative viruses were able to infect and replicate in Vero cells, but that the maximal plaque diameters were markedly reduced, to less than 20 % of wildtype plaque diameters (Fig. 6). Infection with the parental or the rescuant virus resulted in wild-type-like appearance of plaques, indicating that the observed phenotype was indeed induced by the gM-deletion. This was additionally corroborated by the fact, that plaque formation of E4ΔgM-w and E4ΔgM-GFP was fully restored on cell line Vero-gM (Data not shown).

c) Penetration experiments. In this experiment, the influence of the EHV-4 gM on entry kinetics of EHV-4 was assessed. 100 PFU of the different viruses, parental EHV-4, the gM repaired virus E4RgM, as well as the gM-deletion mutants and E4ΔgM-GFP (see Figure 3), were allowed to adsorb at 4°C to Vero cells in 6-well plates. After 90 min, the respective inocula were replaced by fresh medium and penetration was initiated by shifting the incubation temperature to 37°C. At different times after the temperature shift - starting immediately (= 0 min) - extracellular infectivity was pH-inactivated by treating cells with a citrate buffer (pH 3.0). Parallel samples were washed accordingly, but the citrate buffer was replaced by PBS, such that at every time point the "adsorbed infectivity" could be compared to the "penetrated infectivity". Numbers of plaques were determined after incubating cells for four days under a methocell overlay.

c) Several sets of experiments were performed: In figure 7A results are depicted for genotypically and phenotypically gM-negative viruses after propagation on non complementing Vero cells, whereas figure 7B represents kinetics of phenotypically complemented E4ΔgM-w and E4ΔgM-GFP, as viruses had been grown on gM-expressing Vero-gM cells.

A mean of 52.8 % (56,7%) of the infectious parental EHV-4 (E4RgM) was protected from extracellular acid treatment at 40 min after initiating penetration (Fig. 7A, open circles) whereas only 33.7 % (E4ΔgM-w - closed rectangles) and 38.5 % (E4ΔgM-GFP closed triangles) of gM negative viruses were protected, yet. At later time points of entry kinetics, the different graphs start to variously overlap and a maximal penetration efficiency between 61.7% and 78.9% is reached after 150 min of penetration time, indicating that a certain assay variability may account for the slight differences observed.
(Fig. 7A). When gM negative viruses had been prepared on complementing Vero-gM cells no difference at all was observed in their penetration efficiency (7B). As opposed to a delay in entry kinetics of gM-negative EHV-1 of about 20 % (strain RacL11; Osterrieder et al., 1996) to up to 40 % (strain KyA; Seyboldt et al., 2000), the effect of deleting gM of EHV-4 has to be noted with reservation. Nevertheless the following conclusions can be drawn: (i) A difference was observed in kinetics of phenotypically complemented to non complemented gM-negative viruses (Fig. 7A-B), but (ii) the influence of deleting gM on EHV-4 penetration is virtually neclectable.

### Example 3: Analysis of horse sera for anti gM antibodies using a gM specific serological test

To state whether gM can be used as a serological marker for distinction of wild type infection versus vaccination with a gM-negative vaccine, several assumptions had to be tested. Primarily it needed to be assessed whether sera of field virus infected horses contain gM-specific antibodies. For initial analysis a Western blot test was chosen, as this system allowed to identify a specific reaction against background reactivity. When using highly neutralizing sera (EHV-1 and/or -4 neutralizing titers between 1: 128 and 256), it was established (data not shown) that lysates of EHV-1 gM expressing cell line ccgM allowed the detection of a specific signal by horse sera and that a dilution of sera to 1:3000 seemed to work best.
Consequently, sera of all 12 foals (6 vaccinates and 6 controls) that had participated in an EHV-1 gM vaccine trial were analyzed for gM reactivity in Western blot. Of each individual horse three different sera were tested: Taken before entering the trial (Pre), 4 weeks after the second vaccination (V2) and two weeks after challenge infection (C), respectively.
In summary by Western blot analyses (Figure 9) it was shown that (i) sera of horses, exhibiting EHV-1 neutralization activity, all tested positive for gM, that (ii) gM reactivity was never detected in any of the samples analyzed before known contact to EHV-1 or after vaccination with the gM-negative EHV-1 and that (iii) after infection of vaccine trial horses with the gM-positive challenge virus, gM was clearly detectable in 10 out of 12 cases. Finally (iv) it was observed that anti EHV-1 antibody titers and the intensity of gM reactivity seemed not to be directly correlated.
Due to the high background reactivity of horse sera, the establishment of serological tests is difficult. Based on indirect immunofluorescence (IIF) data obtained in horse sera, it was confirmed that either an indirect or a blocking test system will have to be established or highly purified gM-polypeptides need to be used in an ELISA test. To this end, an ELISA was established as follows. Either purified gM polypeptides or the complete gM was immobilised onto the solid support of a 96 well plate which wascoated to ensure good attachment of the capturing protein. For the assay, unspecific binding sites were be blocked by either dry milk or similar substances to prevent unspecific binding. Following this, the plastic surface was rinsed with an appropriate washing buffer (e.g., PBS-Tween) to remove excess blocking agent. Then the test samples were added to selected wells and the ELISA plate was incubated at 37 °C according to standardised methods, allowing antibodies in the test sample to bind to the immobilized capturing protein. In the next step, the wells of the ELISA plate were washed thoroughly by several times rinsing with washing buffer, followed by the addition of a suitable anti-horse antibody coupled to an enzyme such as HRP (horse radish peroxidase). The detection of bound antibody/HRP conjugate was finally performed by adding a suitable substrate, followed by photometric read-out of optical density of individual wells. The value obtained was be compared to positive and negative controls run in the same assay.

### Example 4: Identification of EHV-4 gM

Although the predicted aminoacid sequence of EHV-4 gM is calculated to be 86.7 % identical to that of EHV-1 gM (Telford et al., 1998), anti EHV-1 gM Mab 13B2 (Allen and Yeargan, 1987) specifically reacts in Western blot with the type-specific protein only (Crabb et al., 1991). To nevertheless identify the EHV-4 homolog in this study, other anti-EHV-1 gM antibodies (Seyboldt et al., 2000; Day, 1999) were tested on purified EHV-4 virions, on lysates of cells infected with EHV-4 or on lysates of Vero-gM cells. The latter being a recombinant cell line developed to synthesize EHV-4 gM under control of the IE-HCMV promoter. The reactivity of all anti- EHV-1 gM monoclonal antibodies against EHV-4 gM was below the detection limit in Western blot, whereas parallel EHV-1 samples were always readily reactive (data not shown). Only the polyclonal antiserum, that had been generated in rabbits against a His-tagged EHV-1 gM derived polypeptide (aminoacid 376 - 450; Seyboldt et al., 2000), reacted strong enough with the heterologous gM to allow the identification of EHV-4 gM (Fig. 10 A). Using this antibody a specific reactivity at an Mr of about 50,000 to 55,000 was observed in purified EHV-4 virions. According to its predicted hydrophobic properties the detected gM-protein aggregated upon boiling. In contrast the form of gM expressed in recombinant Vero-gM cells mainly run at an Mr of about 46,000 to 48,000, indicating that the gM-proteins of EHV-4 are processed similarly as has been shown for EHV-1 (Osterrieder et al., 1997; Rudolph and Osterrieder, 2002).

Several experiments were conducted to analyze the phenotype of the gM-deletion in EHV-4. To compare expression of other glycoproteins, lysates of Vero cells infected with EHV-4, E4RgM, E4ΔgM-w or E4ΔgM-GFP were subjected to Western blot analysis. It is demonstrated that the deletion of gM did not influence the production of the late proteins gB or gD, indicating that early steps in virus replication were not substantially affected by the deletion.
In another experiment it could be demonstrated by analysis of virion preparations of wildtype, repaired or both gM-deleted EHV-4, that no gM reactivity at all was detectable within gM-negative viruses, whereas the protein was readily reactive in control virions. The presence of virions in the respective preparation was shown in a parallel blot probing against gB (Fig. 10B).

### References

Allen, G.P., Yeargan, M., Costa, L.R.R. and Cross, R., 1995. Major histocompatibility complex class I-restricted cytotoxic T-lymphocyte responses in horses infected with equine herpesvirus 1. J. Virol. 69, 606-612.
Allen, G.P.and Yeargan, M.R., 1987. Use of λgt11 and monoclonal antibodies to map the genes for the six major glycoproteins of equine herpesvirus1. J. Virol. 61, 2454-2461.
Awan, A.R., Chong, Y.-C. and Field, H.J., 1990. The pathogenesis of equine herpesvirus type 1 in the mouse: A new model for studying host responses to the infection. J. Gen. Virol. 71, 1131-1140.
Baines, J.D. and Roizman, B., 1991. The open reading frames UL3, UL4, UL10 and UL16 are dispensable vor the replication of herpes simplex virus 1 in cell culture. J. Virol. 65, 938-944.
Baines, J.D. and Roizman, B., 1993. The UL10 gene of herpes simplex virus 1 encodes a novel viral glycoprotein, gM, which is present in the virion and in the plasma membrane of infected cells. J. Virol. 67, 1441-1452.
Chalfie M, Tu Y, Euskirchen G, Ward WW, Prasher DC, 1994. Green fluorescent protein as a marker for gene expression. Science 263, 802-805.
Crabb, B.S.; Allen, G.P., Studdert, M.J., 1991. Characterization of the major glycoproteins of equine herpesviruses 4 and 1 and asinine herpesvirus 3 using monoclonal antibodies. J.Gen. Virol. 72, 2075-82.
Day, L. 1999. Characterization of selected glycoproteins of equine herpesvirus-1: immune responses in the murine model. Ph.D. thesis. University of Leeds, Leeds, United Kingdom.
Flowers, C.C. and O'Callaghan, D.J., 1992. The equine herpesvirus type 1 (EHV-1) homolog of herpes simplex virus type 1 US9 and the nature of a major deletion wethin the unique short segment of the EHV-1 KyA strain genome. Virology 190, 307-315.
Hübert, P.H., Birkenmaier, S., Rziha, H.J. and Osterrieder, N., 1996. Alterations in the equine herpesvirus type-1 (EHV-1) strain RacH during attenuation. J. Vet. Med. B 43, 1-14.
Kyhse-Andersen, J., 1984. Electroblotting of multiple gels: a simple apparatus without tank for rapid transfer of proteins from polyacrylamide gels to nitrocellulose. J. Biochem. Biophys. Methods 10, 203-210.
Laemmli, U.K., 1970. Cleavage of structural proteins during the assembly of the head of bacteriophage T4. Nature 227, 680-685.
MacLean, C.A., Robertson, L.M. and Jamieson, F.E., 1993. Characterization of the UL10 gene product of herpes simplex virus type 1 and investigation of ist role in vivo. J. Gen. Virol. 74, 975-983.
Malik, A.K., Martinez, R., Muncy, L., Carmichael, E.P. and Weller, S.K., 1992. Genetic analysis of the herpes simplex virus type 1 UL9 gene: isolation of a LacZ insertion mutant and expression in eukaryotic cells. Virology 190(2), 702-715.
Mayr, A., Pette, J., Petzoldt, K. and Wagener, K., 1968. Untersuchungen zur Entwicklung eines Lebendimpfstoffes gegen die Rhinopneumonitis (Stutenabort) der Pferde. J. Vet. Med. B 15, 406-418.
Neubauer, A., Beer, M., Brandmüller, C., Kaaden, O.-R. and Osterrieder, N., 1997. Equine herpesvirus 1 mutants devoid of glycoprotein B or M are apathogenic for mice but induce protection against challenge infection. Virology 239, 36-45.
Osterrieder, N., Wagner, R., Brandmüller, C., Schmidt, P., Wolf, H. and Kaaden, O.-R., 1995. Protection against EHV-1 challenge infection in the murine model after vaccination with various formulations of recombinant glycoprotein gp14 (gB). Virology 208, 500-510.
Osterrieder, N., Neubauer, A., Brandmüller, C., Braun, B., Kaaden, O.-R. and Baines, J.D., 1996. The equine herpesvirus 1 glycoprotein gp21/22a, the herpes simplex virus type 1 gM homolog, is involved in virus penetration and cell-to-cell spread of virions. Journal of virology, June 1996, p. 4110-4115.
Osterrieder, N.; Neubauer, A.; Fakler, B.; Brandmüller, C.; Seyboldt, C.; Kaaden, O.R.; Baines, J.D., 1997. Synthesis and processing of the equine herpesvirus 1 glykoprotein M. Virology 232, 230-239.
Pilling, A., Davison, A.J., Telford, E.A.R. and Meredith, D.M., 1994. The equine herpesvirus type 1 glycoprotein homologous to herpes simplex virus type 1 glycoprotein M is a major constituent of the virus particle. J. Gen. Virol. 75, 439-442.
Rudolph, J.; Seyboldt, C.; Granzow, H.; Osterrieder, N., 2002. The gene 10 (UL49.5) product of equine herpesvirus 1 is necessary and sufficient for functional processing of glycoprotein M. J. Virology 76, 2952-2963.
Sambrook, J., Fritsch, D.F. and Maniatis, T., 1989. Molecular Cloning: A laboratory manual. 2nd ed. Cold Spring Harbor Laboratory Press, Cold Spring Harbor, NY.
Seyboldt, C., 2000. Structural and functional analysis of the equine herpesvirus type 1 glycoprotein M. Doctoral thesis, Ludwig-Maximilians-University, Munich, Germany.
Seyboldt, C.; Granzow, H.; Osterrieder, N. 2000. Equine herpesvirus 1 (EHV-1) Glycoprotein M: Effect of deletions of transmembrane domains. Virology 278, 477-489.
Stokes, A., Alber, D.G., Greensill, J., Amellal, B., Carvalho, R., Taylor, L.A., Doel, T.R., Killington, R.A., Halliburton, I.W. and Meredith, D.M., 1996. The expression of the proteins of equine herpesvirus 1 which share homology with herpes simplex virus 1 glycoproteins H and L. Virus Res. 40, 91-107.
Telford, E.A.R., Watson, M.S., McBride, K. and Davison, A.J., 1992. The DNA sequence of equine herpesvirus-1. Virology 189, 304-316.
Telford, E.A.R., Watson, M.S., Perry, J., Cullinane, A.A. and Davison, A.J., 1998. The DNA sequence of equine herpesvirus-4. Journal of Gen. Virol. 79, 1197-1203.
Tewari, D., Whalley, J.M., Love, D.N. and Field, H.J., 1994. Characterisation of immune responses to baculovirus expressed equine herpesvirus type 1 glycoproteins D and H in a murine model. J. Gen. Virol. 75, 1735-1741.

## Claims

1. An Equine Herpes Virus wherein the protein gM is absent, **characterized in that** said Equine Herpes Virus is free of heterologous elements.

2. The Equine Herpes Virus according to claim 1, **characterized in that** the gene coding for the protein gM is deleted.

3. The Equine Herpes Virus according to claim 1 or 2, obtainable by a method comprising the steps of
a) isolating a wild-type Equine Herpes Virus;
b) establishing a plasmid encoding the Equine Herpes Virus gM gene, optionally with flanking sequences;
c) generating a complementing cell line expressing gM or parts thereof;
d) establishing an Equine Herpes Virus carrying a GFP-encoding cassette insert in its gM coding sequence by co-transfecting the complementing cell line of step b) with Equine Herpes Virus-nucleic acid and a plasmid encoding gM which is interrupted by a GFP-encoding cassette insert;
e) deleting the GFP-encoding cassette; and
f) selecting for the Equine Herpes Virus clones wherein the GFP-encoding cassette is successfully deleted.

4. The Equine Herpes Virus according to any one of claims 1 to 3, **characterized in that** it is Equine Herpes Virus-1.

5. The Equine Herpes Virus-1 according to claim 4, **characterized in that** 850-1100 bp of the gM open reading frame are deleted.

6. The Equine Herpes Virus-1 according to claims 4 or 5, **characterized in that** the entire gM coding sequence is deleted except for 150-200 bp of the coding sequence for the C-terminal portion and except for 150-250 bp of the coding sequence for the N-terminal portion.

7. The Equine Herpes Virus-1 according to any one of claims 4 to 6, **characterized in that** nucleotides 93268-93318 to 94222-94322 of the gM coding sequence are deleted, wherein the nucleotide positions refer to the nucleotide positions of the Equine Herpes Virus sequence as described in Telford 1992.

8. The Equine Herpes Virus-1 according to any one of claims 5 to 7, **characterized in that** nucleotides 93268 to 94322 of the gM coding sequence are deleted, wherein the nucleotide positions refer to the nucleotide positions of the Equine Herpes Virus sequence as described in Telford 1992.

9. The Equine Herpes Virus-1 according to any one of claims 5 to 8, **characterized in that** the entire gM coding sequence is deleted except for 184 bp of the coding sequence for the C-terminal portion and except for 209 bp of the coding sequence for the N-terminal portion.

10. The Equine Herpes Virus-1 according to any one of claims 5 to 9, **characterized in that** nucleotides 94263 to 93302 of the gM coding sequence are deleted, wherein the nucleotide positions refer to the nucleotide positions of the EHV sequence as described in Telford 1992.

11. The Equine Herpes Virus-1 according to any one of claims 5 to 10, **characterized in that** it is a recombinant variant based on strain RacH of EHV-1.

12. An Equine Herpes Virus-1 **characterized in that** it is isolate HΔgM-w deposited at the ECACC/CAMR on October 16, 2002 with the accession number 02101663.

13. The Equine Herpes Virus according to any one of claims 1 to 3, **characterized in that** it is Equine Herpes Virus-4.

14. The Equine Herpes Virus-4 according to claim 13, **characterized in that** 900-1150 bp of the gM open reading frame are deleted.

15. The Equine Herpes Virus-4 according to claims 13 or 14, **characterized in that** the entire gM coding sequence is deleted except for 0-50 bp of the coding sequence for the C-terminal portion and except for 150-250 bp of the coding sequence for the N-terminal portion.

16. The Equine Herpes Virus-4 according to any one of claims 13 to 15, **characterized in that** nucleotides 92681-92731 to 93765-93865 of the gM coding sequence are deleted, wherein the nucleotide positions refer to the nucleotide positions of the Equine Herpes Virus sequence as described in Telford 1998.

17. The Equine Herpes Virus-4 according to any one of claims 13 to 16, **characterized in that** nucleotides 92681 to 93865 of the gM coding sequence are deleted, wherein the nucleotide positions refer to the nucleotide positions of the Equine Herpes Virus sequence as described in Telford 1998.

18. The Equine Herpes Virus-4 according to any one of claims 13 to 17, **characterized in that** the entire gM coding sequence is deleted except for 34 bp of the coding sequence for the C-terminal portion and except for 209 bp of the coding sequence for the N-terminal portion.

19. The Equine Herpes Virus-4 according to any one of claims 13 to 18, **characterized in that** nucleotides 92715 to 93824 of the gM coding sequence are deleted, wherein the nucleotide positions refer to the nucleotide positions of the Equine Herpes Virus sequence as described in Telford 1998.

20. An Equine Herpes Virus-4 **characterized in that** it is isolate E4ΔgM-w deposited at the ECACC/CAMR on January 14, 2003 with the accession number 03011401.

21. A nucleic acid coding for an Equine Herpes Virus according to any one of claims 1 to 20.

22. A vaccine preparation comprising an Equine Herpes Virus according to any one of claims 1 to 20.

23. A vaccine preparation comprising a nucleic acid according to claim 21.

24. Use of Equine Herpes Virus according to any one of claims 1 to 20 in the manufacture of a medicament for the prophylaxis and/or treatment of Equine Herpes Virus-Infections.

25. Use of a nucleic acid according to claim 21 in the manufacture of a medicament for the prophylaxis and/or treatment of Equine Herpes Virus-Infections.

26. A vaccine preparation comprising at least one Equine Herpes Virus-1 according to claims 1 to 12 and one Equine Herpes Virus-4 according to claims 13 to 20.

27. Use of at least one Equine Herpes Virus-1 according to claims 1 to 12 and one Equine Herpes Virus-4 according to claims 13 to 20 in the manufacture of a medicament for treatment of Equine Herpes Virus-Infections.

28. Method for obtaining a recombinant Equine Herpes Virus according to claim 1, comprising the steps of
a) isolating a wild-type Equine Herpes Virus;
b) establishing a plasmid encoding the Equine Herpes Virus gM gene, optionally with flanking sequences;
c) generating a complementing cell line expressing gM or parts thereof;
d) establishing an Equine Herpes Virus carrying a GFP-encoding cassette insert in its gM coding sequence by co-transfecting the complementing cell line of step b) with EHV-nucleic acid and a plasmid encoding gM which is interrupted by a GFP-encoding cassette insert;
e) deleting the GFP-encoding cassette; and
f) selecting for the EHV clones wherein the GFP-encoding cassette is successfully deleted.

29. A gM complementing cell line **characterized in that** it is VERO GM deposited at the ECACC/CAMR on January 28, 2003 with the accession number 03012801.

## Patentansprüche

1. Equines Herpes-Virus, in dem das Protein gM fehlt, **dadurch gekennzeichnet, dass** das equine Herpes-Virus frei von heterologen Elementen ist.

2. Equines Herpes-Virus nach Anspruch 1, **dadurch gekennzeichnet, dass** das für das Protein gM kodierende Gen deletiert ist.

3. Equines Herpes-Virus nach Anspruch 1 oder 2, erhältlich durch ein Verfahren, das die folgenden Stufen umfasst:
a) das Isolieren eines equinen Wildtyp-Herpes-Virus;
b) das Bilden eines Plasmids, das für das equine Herpes-Virus-gM-Gen kodiert, gegebenenfalls mit flankierenden Sequenzen;
c) das Erzeugen einer komplementierenden Zelllinie, die gM oder Teile davon exprimiert;
d) das Bilden eines equinen Herpes-Virus, das in seiner gM-Kodierungssequenz ein für GFP kodierendes Kassetteninsert trägt, durch Kotransfizieren der komplementierenden Zelllinie von Stufe b) mit equiner Herpes-Virus-Nucleinsäure und einem Plasmid, das für gM kodiert, das durch ein für GFP kodierendes Kassetteninsert unterbrochen ist;
e) das Deletieren der für GFP kodierenden Kassette; und
f) das Auswählen von equinen Herpes-Virus-Klonen, bei denen die für GFP kodierende Kassette in erfolgreicher Weise deletiert ist.

4. Equines Herpes-Virus nach einem der Ansprüche 1 bis 3, **dadurch gekennzeichnet, dass** es sich um equines Herpes-Virus-1 handelt.

5. Equines Herpes-Virus-1 nach Anspruch 4, **dadurch gekennzeichnet, dass** 850-1100 bp des offenen gM-Leserasters deletiert sind.

6. Equines Herpes-Virus-1 nach Anspruch 4 oder 5, **dadurch gekennzeichnet, dass** die gesamte gM-Kodierungssequenz deletiert ist, mit Ausnahme von 150-200 bp der Kodierungssequenz für den C-terminalen Bereich und mit Ausnahme von 150-250 bp der Kodierungssequenz für den N-terminalen Bereich.

7. Equines Herpes-Virus-1 nach einem der Ansprüche 4 bis 6, **dadurch gekennzeichnet, dass** die Nucleotide 93 268-93 318 bis 94 222-94 322 der gM-Kodierungssequenz deletiert sind, wobei sich die Nucleotidpositionen auf die Nucleotidpositionen der in Telford 1992 beschriebenen equinen Herpes-Virus-Sequenz beziehen.

8. Equines Herpes-Virus-1 nach einem der Ansprüche 5 bis 7, **dadurch gekennzeichnet, dass** die Nucleotide 93 268 bis 94322 der gM-Kodierungssequenz deletiert sind, wobei sich die Nucleotidpositionen auf die Nucleotidpositionen der in Telford 1992 beschriebenen equinen Herpes-Virus-Sequenz beziehen.

9. Equines Herpes-Virus-1 nach einem der Ansprüche 5 bis 8, **dadurch gekennzeichnet, dass** die gesamte gM-Kodierungssequenz deletiert ist, mit Ausnahme von 184 bp der Kodierungssequenz für den C-terminalen Bereich und mit Ausnahme von 209 bp der Kodierungssequenz für den N-terminalen Bereich.

10. Equines Herpes-Virus-1 nach einem der Ansprüche 5 bis 9, **dadurch gekennzeichnet, dass** Nucleotide 94 263 bis 93 302 der gM-Kodierungssequenz deletiert sind, wobei sich die Nucleotidpositionen auf die Nucleotidpositionen der in Telford 1992 beschriebenen equinen Herpes-Virus-Sequenz beziehen.

11. Equines Herpes-Virus-1 nach einem der Ansprüche 5 bis 10, **dadurch gekennzeichnet, dass** es sich um eine rekombinante Variante auf der Basis des Stammes RacH von EHV-1 handelt.

12. Equines Herpes-Virus-1, **dadurch gekennzeichnet, dass** es sich um das Isolat HΔgM-w handelt, das bei ECACC/CAMR am 16. Oktober 2002 unter der Hinterlegungsnummer 02101663 hinterlegt worden ist.

13. Equines Herpes-Virus nach einem der Ansprüche 1 bis 3, **dadurch gekennzeichnet, dass** es sich um equines Herpes-Virus-4 handelt.

14. Equines Herpes-Virus-4 nach Anspruch 13, **dadurch gekennzeichnet, dass** 900-1 150 bp des offenen gM-Leserasters deletiert sind.

15. Equines Herpes-Virus-4 nach Anspruch 13 oder 14, **dadurch gekennzeichnet, dass** die gesamte gM-Kodierungssequenz deletiert ist, mit Ausnahme von 0-50 bp der Kodierungssequenz für den C-terminalen Bereich und mit Ausnahme von 150-250 bp der Kodierungssequenz für den N-terminalen Bereich.

16. Equines Herpes-Virus-4 nach einem der Ansprüche 13 bis 15, **dadurch gekennzeichnet, dass** die Nucleotide 92681-92731 bis 93765-93865 der gM-Kodierungssequenz deletiert sind, wobei sich die Nucleotidpositionen auf die Nucleotidpositionen der in Telford 1998 beschriebenen equinen Herpes-Virus-Sequenz beziehen.

17. Equines Herpes-Virus-4 nach einem der Ansprüche 13 bis 16, **dadurch gekennzeichnet, dass** die Nucleotide 92 681 bis 93 865 der gM-Kodierungssequenz deletiert sind, wobei sich die Nucleotidpositionen auf die Nucleotidpositionen der in Telford 1998 beschriebenen equinen Herpes-Virus-Sequenz beziehen.

18. Equines Herpes-Virus-4 nach einem der Ansprüche 13 bis 17, **dadurch gekennzeichnet, dass** die gesamte gM-Kodierungssequenz deletiert ist, mit Ausnahme von 34 bp der Kodierungssequenz für den C-terminalen Bereich und mit Ausnahme von 209 bp der Kodierungssequenz für den N-terminalen Bereich.

19. Equines Herpes-Virus-4 nach einem der Ansprüche 13 bis 18, **dadurch gekennzeichnet, dass** die Nucleotide 92 715 bis 93 824 der gM-Kodierungssequenz deletiert sind, wobei sich die Nucleotidpositionen auf die Nucleotidpositionen der in Telford 1998 beschriebenen equinen Herpes-Virus-Sequenz beziehen.

20. Equines Herpes-Virus-4, **dadurch gekennzeichnet, dass** es sich um das Isolat E4ΔgM-w handelt, das bei der ECACC/CAMR am 14. Januar 2003 unter der Hinterlegungsnummer 03011401 hinterlegt worden ist.

21. Nucleinsäure, kodierend für ein equines Herpes-Virus nach einem der Ansprüche 1 bis 20.

22. Impfstoffzubereitung, umfassend ein equines Herpes-Virus nach einem der Ansprüche 1 bis 20.

23. Impfstoffzubereitung, umfassend eine Nucleinsäure nach Anspruch 21.

24. Verwendung von equinem Herpes-Virus nach einem der Ansprüche 1 bis 20 bei der Herstellung eines Arzneimittels zur Prophylaxe und/oder Behandlung von equinen Herpes-Virus-Infektionen.

25. Verwendung einer Nucleinsäure nach Anspruch 21 bei der Herstellung eines Arzneimittels zur Prophylaxe und/oder Behandlung von equinen Herpes-Virus-Infektionen.

26. Impfstoffzubereitung, umfassend mindestens ein equines Herpes-Virus-1 nach Anspruch 1 bis 12 und ein equines Herpes-Virus-4 nach Anspruch 13 bis 20.

27. Verwendung mindestens eines equinen Herpes-Virus-1 nach Anspruch 1 bis 12 und eines equinen Herpes-Virus-4 nach Anspruch 13 bis 20 bei der Herstellung eines Arzneimittels zur Behandlung von equinen Herpes-Virus-Infektionen.

28. Verfahren zur Gewinnung eines rekombinanten equinen Herpes-Virus nach Anspruch 1, umfassend die folgenden Stufen:
a) das Isolieren eines equinen Wildtyp-Herpes-Virus;
b) das Bilden eines Plasmids, das für das equine Herpes-Virus-gM-Gen kodiert, gegebenenfalls mit flankierenden Sequenzen;
c) das Erzeugen einer komplementierenden Zelllinie, die gM oder Teile davon exprimiert;
d) das Bilden eines equinen Herpes-Virus, das in seiner gM-Kodierungssequenz ein für GFP kodierendes Kassetteninsert trägt, durch Cotransfizieren der komplementierenden Zelllinie von Stufe b) mit equiner Herpes-Virus-Nucleinsäure und einem Plasmid, das für gM kodiert, das durch ein für GFP kodierendes Kassetteninsert unterbrochen ist;
e) das Deletieren der für GFP kodierenden Kassette; und
f) das Auswählen von equinen Herpes-Virus-Klonen, bei denen die für GFP kodierende Kassette in erfolgreicher Weise deletiert ist.

29. gM-komplementierende Zelllinie, **dadurch gekennzeichnet, dass** es sich um VERO GM handelt, die bei der ECACC/CAMR am 28.Januar 2003 unter der Hinterlegungsnummer 03012801 hinterlegt worden ist.

## Revendications

1. Herpèsvirus équin où la protéine gM est absente, **caractérisé en ce que** ledit herpèsvirus équin est dépourvu d'éléments hétérologues.

2. Herpèsvirus équin selon la revendication 1 **caractérisé en ce que** le gène codant la protéine gM est délété.

3. Herpèsvirus équin selon la revendication 1 ou 2 pouvant être obtenu par un procédé comprenant les étapes de
a) isoler un herpèsvirus équin de type sauvage ;
b) établir un plasmide codant le gène de gM de l'herpèsvirus équin, éventuellement avec des séquences flanquantes ;
c) générer une lignée cellulaire de complémentation exprimant gM ou des parties de celle-ci ;
d) établir un herpèsvirus équin portant un insert de cassette codant GFP dans sa séquence codant gM par co-transfection de la lignée cellulaire de complémentation de l'étape b) avec l'acide nucléique de l'herpèsvirus équin et un plasmide codant gM qui est interrompu par un insert de cassette codant GFP ;
e) déléter la cassette codant GFP ; et
f) opérer une sélection pour les clones d'herpèsvirus équin où la cassette codant GFP est délétée avec succès.

4. Herpèsvirus équin selon l'une quelconque des revendications 1 à 3 **caractérisé en ce que** c'est un herpèsvirus équin-1.

5. Herpèsvirus équin-1 selon la revendication 4 **caractérisé en ce que** 850-1100 pb du cadre de lecture ouvert de gM sont délétés.

6. Herpèsvirus équin-1 selon la revendication 4 ou 5 **caractérisé en ce que** toute la séquence codant gM est délétée à l'exception de 150-200 pb de la séquence codante pour la partie C-terminale et à l'exception de 150-250 pb de la séquence codante pour la partie N-terminale.

7. Herpèsvirus équin-1 selon l'une quelconque des revendications 4 à 6 **caractérisé en ce que** les nucléotides 93268-93318 à 94222-94322 de la séquence codant gM sont délétés, où les positions de nucléotides se réfèrent aux positions de nucléotides de la séquence d'herpèsvirus équin décrite dans Telford 1992.

8. Herpèsvirus équin-1 selon l'une quelconque des revendications 5 à 7 **caractérisé en ce que** les nucléotides 93268 à 94322 de la séquence codant gM sont délétés, où les positions de nucléotides se réfèrent aux positions de nucléotides de la séquence d'herpèsvirus équin décrite dans Telford 1992.

9. Herpèsvirus équin-1 selon l'une quelconque des revendications 5 à 8 **caractérisé en ce que** toute la séquence codant gM est délétée à l'exception de 184 pb de la séquence codante pour la partie C-terminale et à l'exception de 209 pb de la séquence codante pour la partie N-terminale.

10. Herpèsvirus équin-1 selon l'une quelconque des revendications 5 à 9 **caractérisé en ce que** les nucléotides 94263 à 93302 de la séquence codant gM sont délétés, où les positions de nucléotides se réfèrent aux positions de nucléotides de la séquence de EHV décrite dans Telford 1992.

11. Herpèsvirus équin-1 selon l'une quelconque des revendications 5 à 10 **caractérisé en ce que** c'est un variant recombinant basé sur la souche RacH de EHV-1.

12. Herpèsvirus équin-1 **caractérisé en ce que** c'est l'isolat HΔgM-w déposé à ECACC/CAMR le 16 octobre 2002 sous le numéro d'ordre 02101663.

13. Herpèsvirus équin selon l'une quelconque des revendications 1 à 3 **caractérisé en ce que** c'est un herpèsvirus équin-4.

14. Herpèsvirus équin-4 selon la revendication 13 **caractérisé en ce que** 900-1150 pb du cadre de lecture ouvert de gM sont délétés.

15. Herpésvirus équin-4 selon la revendication 13 ou 14 **caractérisé en ce que** toute la séquence codant gM est délétée à l'exception de 0-50 pb de la séquence codante pour la partie C-terminale et à l'exception de 150-250 pb de la séquence codante pour la partie N-terminale.

16. Herpèsvirus équin-4 selon l'une quelconque des revendications 13 à 15 **caractérisé en ce que** les nucléotides 92681-92731 à 93765-93865 de la séquence codant gM sont délétés, où les positions de nucléotides se réfèrent aux positions de nucléotides de la séquence d'herpèsvirus équin décrite dans Telford 1998.

17. Herpèsvirus équin-4 selon l'une quelconque des revendications 13 à 16 **caractérisé en ce que** les nucléotides 92681 à 93865 de la séquence codant gM sont délétés, où les positions de nucléotides se réfèrent aux positions de nucléotides de la séquence d'herpèsvirus équin décrite dans Telford 1998.

18. Herpèsvirus équin-4 selon l'une quelconque des revendications 13 à 17 **caractérisé en ce que** toute la séquence codant gM est délétée à l'exception de 34 pb de la séquence codante pour la partie C-terminale et à l'exception de 209 pb de la séquence codante pour la partie N-terminale.

19. Herpèsvirus équin-4 selon l'une quelconque des revendications 13 à 18 **caractérisé en ce que** les nucléotides 92715 à 93824 de la séquence codant gM sont délétés, où les positions de nucléotides se réfèrent aux positions de nucléotides de la séquence d'herpèsvirus équin décrite dans Telford 1998.

20. Herpèsvirus équin-4 **caractérisé en ce que** c'est l'isolat E4ΔgM-w déposé auprès de ECACC/CAMR le 14 janvier 2003 sous le numéro d'ordre 03011401.

21. Acide nucléique codant un herpèsvirus équin selon l'une quelconque des revendications 1 à 20.

22. Préparation vaccinale comprenant un herpèsvirus équin selon l'une quelconque des revendications 1 à 20.

23. Préparation vaccinale comprenant un acide nucléique selon la revendication 21.

24. Utilisation d'un herpèsvirus équin selon l'une quelconque des revendications 1 à 20 dans la fabrication d'un médicament pour la prophylaxie et/ou le traitement des infections à herpèsvirus équin.

25. Utilisation d'un acide nucléique selon la revendication 21 dans la fabrication d'un médicament pour la prophylaxie et/ou le traitement des infections à herpèsvirus équin.

26. Préparation vaccinale comprenant au moins un herpèsvirus équin-1 selon les revendications 1 à 12 et un herpèsvirus équin-4 selon les revendications 13 à 20.

27. Utilisation d'au moins un herpèsvirus équin-1 selon les revendications 1 à 12 et un herpèsvirus équin-4 selon les revendications 13 à 20 dans la fabrication d'un médicament pour le traitement des infections à herpèsvirus équin,

28. Procédé pour obtenir un herpèsvirus équin recombinant selon la revendication 1 comprenant les étapes de
a) isoler un herpèsvirus équin de type sauvage ;
b) établir un plasmide codant le gène de gM de l'herpèsvirus équin, éventuellement avec des séquences flanquantes ;
c) générer une lignée cellulaire de complémentation exprimant gM ou des parties de celle-ci ;
d) établir un herpèsvirus équin portant un insert de cassette codant GFP dans sa séquence codant gM par co-transfection de la lignée cellulaire de complémentation de l'étape b) avec l'acide nucléique de EHV et un plasmide codant gM qui est interrompu par un insert de cassette codant GFP ;
e) déléter la cassette codant GFP ; et
f) opérer une sélection pour les clones de EHV où la cassette codant GFP est délétée avec succès.

29. Lignée cellulaire de complémentation de gM **caractérisée en ce que** c'est VERO GM déposée auprès de ECACC/CAMR le 28 janvier 2003 sous le numéro d'ordre 03012801.
